# EUROPEAN PATENT SPECIFICATION

(11) **EP 1 667 730 B1**
(45) Date of publication and mention of the grant of the patent: **26.06.2013**
(21) Application number: 04781986.7
(22) Date of filing: 20.08.2004
(51) Int. Cl.: A61K 48/00, C12N 15/85, C12N 15/86, C07H 21/04, C12N 9/00

(54) **COMPOSITIONS AND METHODS FOR TREATING INFLAMMATORY LUNG DISEASE**
ZUSAMMENSETZUNGEN UND VERFAHREN ZUR BEHANDLUNG VON ENTZÜNDLICHER LUNGENERKRANKUNG
COMPOSITIONS ET PROCEDES POUR LE TRAITEMENT DE LA MALADIE PULMONAIRE INFLAMMATOIRE

(30) Priority: 20.08.2003 US 496555 P; 20.08.2003 US 496625 P; 03.09.2003 US 499768 P; 17.02.2004 US 545226 P
(43) Date of publication of application: 14.06.2006
(60) Divisional application: 10188265.2
(73) Proprietor: University of Miami, Miami, FL 33136 (US)
(72) Inventor: PODACK, Eckhard, R., Cocunut Grove, FL 33133 (US); FANG, Lei, Germantown, MD 20874 (US)
(74) Representative: Rickard, David John
(86) International application number: PCT/US2004/027407
(87) International publication number: WO 2005/018571

(56) References cited:
- WO-A-00/64465
- WO-A-01/35995
- WO-A-03/000286
- WO-A-03/043583
- WO-A1-99/43839
- US-A1- 2003 092 044
- US-A1- 2003 129 189
- US-B1- 6 534 061
- MIGONE THI-SAU ET AL: "TL1A is a TNF-like ligand for DR3 and TR6/DcR3 and functions as a T cell costimulator" IMMUNITY, CELL PRESS, US, vol. 16, no. 3, March 2002 (2002-03), pages 479-492, XP002397922 ISSN: 1074-7613
- MATTES J ET AL: "IL-13 induces airways hyperreactivity independently of the IL-4R alpha chain in the allergic lung." JOURNAL OF IMMUNOLOGY (BALTIMORE, MD. : 1950) 1 AUG 2001, vol. 167, no. 3, 1 August 2001 (2001-08-01), pages 1683-1692, XP002453199 ISSN: 0022-1767
- AKBARI OMID ET AL: "Essential role of NKT cells producing IL-4 and IL-13 in the development of allergen-induced airway hyperreactivity." NATURE MEDICINE, vol. 9, no. 5, May 2003 (2003-05), pages 582-588, XP002453200 ISSN: 1078-8956
- WANG EDDIE C Y ET AL: "DR3 regulates negative selection during thymocyte development" MOLECULAR AND CELLULAR BIOLOGY, AMERICAN SOCIETY FOR MICROBIOLOGY, WASHINGTON, US, vol. 21, no. 10, May 2001 (2001-05), pages 3451-3461, XP002208363 ISSN: 0270-7306
- NAM SANG-YUN ET AL: "CD30 deficiency diminishes airway eosinophilia in a mouse model of pulmonary inflammation." FASEB JOURNAL, vol. 17, no. 7, 14 April 2003 (2003-04-14), page C14, XP009093136 & 90TH ANNIVERSARY ANNUAL MEETING OF THE AMERICAN ASSOCIATION OF IMMUNOLOGISTS; DENVER, CO, USA; MAY 06-10, 2003 ISSN: 0892-6638
- PURELLO-D'AMBROSIO F ET AL: "Effect of fluticasone propionate on soluble CD30 release in patients with severe allergic asthma" JOURNAL OF INVESTIGATIONAL ALLERGOLOGY AND CLINICAL IMMUNOLOGY, vol. 10, no. 5, September 2000 (2000-09), pages 283-285, XP009093129 ISSN: 1018-9068
- WILLS-KARP M ET AL: "INTERLEUKIN-13: CENTRAL MEDIATOR OF ALLERGIC ASTHMA" SCIENCE, AMERICAN ASSOCIATION FOR THE ADVANCEMENT OF SCIENCE,, US, vol. 282, 18 December 1998 (1998-12-18), pages 2258-2260, XP002948381 ISSN: 0036-8075
- GRUENIG G ET AL: "REQUIREMENT FOR IL-13 INDEPENDENTLY OF IL-4 IN EXPERIMENTAL ASTHMA" SCIENCE, AMERICAN ASSOCIATION FOR THE ADVANCEMENT OF SCIENCE,, US, vol. 282, 18 December 1998 (1998-12-18), pages 2261-2263, XP002948380 ISSN: 0036-8075
- LI L ET AL: "Effects of Th2 cytokines on chemokine expression in the lung: IL-13 potently induces eotaxin expression by airway epithelial cells." JOURNAL OF IMMUNOLOGY (BALTIMORE, MD. : 1950) 1 MAR 1999, vol. 162, no. 5, 1 March 1999 (1999-03-01), pages 2477-2487, XP002460824 ISSN: 0022-1767
- HARLIN, H. ET AL.: 'TCR-Independent CD30 Signaling Induces IL-13 Production Via a TNF Receptor-Associated Factor/p38 Mitogen-Activated Protein Kinase-Dependent Mechanism' J. IMMUNOLOGY vol. 169, September 2002, pages 2451 - 2460, XP002985842
- DEL PRETE, G. ET AL.: 'CD30-mediated Signaling Promotes the Development of Human T helper Type 2 like T cells' J. EXP. MED. vol. 182, December 1995, pages 1655 - 1661, XP002985843
- LEONARD, C. ET AL.: 'Allergen-induced CD30 expression on T cells of atopic asthmatics' CLINICAL AND EXP. ALLERGY vol. 27, July 1997, pages 780 - 786, XP002985844

## Description

The invention relates to the fields of medicine and immunology. More particularly, the invention relates to antibodies for modulating responses involved in asthma.

### BACKGROUND OF THE INVENTION

Asthma is an episodic reactive airway disease characterized by hypersensitivity to allergens and other stimuli, edema, airway constriction, and mucus overproduction. It is prevalent in industrialized countries, occurring in about 4-5% of the U.S. population. Each year it is responsible for about 3 million physician office visits, a couple hundred thousand hospitalizations, and several thousand deaths. Lasley, M., Pediatrics in Review, 24:222, 2003. Its economic impact is estimated at almost $3 billion per year. Id.

The hallmark of asthma is tracheobronchial inflammation triggered by various different stimuli including allergens, exercise, infections, and emotional stress. Exacerbating the situation, this inflammation makes the airway hyper-responsive to the triggering stimuli. Untreated, chronic airway inflammation can lead to irreversible anatomical changes and permanent loss of pulmonary function. Although the biochemical and cellular mechanisms responsible for triggering the inflammation associated with asthma are not completely understood, the asthmatic airway tissue itself is characterized by increased levels of inflammatory mediators such as histamine, bradykinin, leukotrienes, platelet-activating factor, prostaglandins thromboxane various cytokines (e.g., IL-4, IL-5, IL-8, and IL-13) and chemokines (e.g., LTB-4 and eotaxin); and infiltration of mast cells, eosinophils, T lymphocytes, platelets, and neutrophils.

Of the cytokines involved in inflammation of the lung, IL-13 has been recognized as a central player in allergic, inflammatory lung disease and airway hyper-reactivity (AHR)(see, for example, Mattes et al., J Immunol 167:1683, 2001; Pinto et al., Blood, 88: 3299-3305, 1996; Pope et al., J Allergy Clin Immunol, 108: 594-601., 2001. For example, studies have shown (i) that IL-13 production by TH2 type CD4 cells is required for airway hyper-reactivity and contraction, mucus overproduction, and goblet cell hyperplasia (Whittaker et al., Am J Respir Cell Mol Biol, 27: 593-602,2002) and (ii) that IL-13 contributes to inflammatory cell infiltration.

T lymphocytes play a central role in regulating immune responses. Helper T cells express the CD4 surface marker and provide help to B cells for antibody production and help CD8 T cells to develop cytotoxic activity. Other CD4 T cells inhibit antibody production and cytotoxicity. T cells regulate the equilibrium between attack of infected or tumorigenic cells and tolerance to the body's cells. Dysregulated immune attack can lead to autoimmunity, while diminished immune responsiveness results in chronic infection and cancer. CD30, as disclosed herein, is a regulator both of the initiation of the T cell response as well as a terminator of the response at a later stage of the immune response.

Death receptor 3 (DR3) (Chinnaiyan et al., Science 274:990, 1996) is a member of the TNF-receptor family (TNFRSF25). It is also known as TRAMP (Bodmer et al., Immunity 6:79, 1997), wsl-1 (Kitson et al., Nature 384:372, 1996), Apo-3 (Marsters et al., Curr Biol 6:1669, 1996), and LARD (Screaton et al., Proc Natl Acad Sci U S A 94:4615, 1997) and contains a typical death domain. Transfection of 293 cells with human DR3 (hDR3) induced apoptosis and activated NF-κB. The cognate ligand for DR3 has recently been identified as TL1A (Migone et al., Immunity 16:479, 2002) and has been shown to have costimulatory activity for DR3 on T cells through the induction of NF-κB and suppression of apoptosis by expression cIAP2 (Wen et al., J Biol Chem 25:25, 2003). TL1A also binds to the decoy receptor 3 (DcR3/TR-6), suggesting that fine-tuning of biological TL1A accessibility is of critical importance. Multiple spliced forms of human DR3 mRNA have been observed, suggesting regulation at the post transcriptional level (Screaton et al., Proc Natl Acad Sci U S A 94:4615, 1997).

Many TNF-receptor family members have the ability to induce cell death by apoptosis or induce costimulatory signals for T cell function. The regulation of these opposing pathways has recently been clarified for TNF-R1, the prototypic death domain-containing receptor that can cause apoptosis or proliferation of receptor positive T cells (Micheau and Tschopp. Cell 114:181, 2003). NF-κB activation by a signaling complex composed of TNF-R1 via TRADD, TRAF2 and RIP induces FLIPL association with a second signaling complex composed of TNFRI, TRADD and FADD, preventing caspase 8 activation as long as the NF-κB signaling persists. DR3 has been shown to be able to induce apoptosis in transfected cells and to induce NF-κB and all three MAP-kinase pathways (Chinnaiyan et al., Science 274:990, 1996; Bodmer et al., Immunity 6:79, 1997; Kitson et al., Nature 384:372,1996; Marsters et al., Curr Biol 6:1669, 1996; Screaton et al., Proc Natl Acad Sci U S A 94:4615, 1997; Wen et al., J Biol Chem 25:25, 2003). Blocking of NF-κB, but not of MAP-kinase and inhibition of protein synthesis resulted in DR3-mediated cell death, suggesting that NF-κB signals mediate anti-apoptotic effects through the synthesis of anti-apoptotic proteins.

Expression of human DR3 is pronounced in lymphoid tissues, mainly in the spleen, lymph nodes, thymus, and small intestine, suggesting an important role for DR3 in lymphocytes. Murine DR3 has been deleted by homologous recombination in embryonic stem cells (Wang et al., Mol Cell Biol 21:3451, 2001). DR3-/- mice show diminished negative selection by anti-CD3 in the thymus but normal negative selection by superantigens and unimpaired positive selection of thymocytes. Mature peripheral T cells were unaffected by DR3 deficiency. Despite a significant amount of preliminary research, the physiological function of DR3 remains poorly characterized.

W00135995 discloses biologically active TR3-specific binding agents and methods for their use. The biologically active TR3-specific binding agents are useful for inhibiting the proliferation of cells expressing TR3. These biologically active agents are particularly useful for treating T-cell mediated diseases such as graft-versus-host disease, organ rejection, tumor growth, autoimmunity, and inflammation.

WO0064465 discloses novel Death Domain Containing Receptor (DR3 and DR3-V1) proteins which are members of the tumor necrosis factor (TNF) receptor family. In particular, isolated nucleic acid molecules are provided encoding the human DR3 and DR3-V1 proteins. DR3 and DR3-V1 polypeptides are also provided, as are vectors, host cells and recombinant methods for producing the same. Further described are screening methods for identifying agonists and antagonists of DR3 and DR3-V1 activity.

US2003129189 discloses human TNF-gamma-alpha and TNF-gamma-beta polypeptides and DNA (RNA) encoding such polypeptides and a procedure for producing such polypeptides by recombinant techniques. Also disclosed are methods for utilizing such polypeptides to inhibit cellular growth, for example in a tumor or cancer, for facilitating wound-healing, to provide resistance against infection, induce inflammatory activities, and stimulating the growth of certain cell types to treat diseases, for example restenosis. Also disclosed are diagnostic methods for detecting a mutation in the TNF-gamma-alpha and TNF-gamma-beta nucleic acid sequences or overexpression of the TNF-gamma-alpha and/or TNF-gamma-beta polypeptides. Antagonists against such polypeptides and their use as a therapeutic to treat cachexia, septic shock, cerebral malaria, inflammation, arthritis and graft-rejection are also disclosed.

Various molecules are therefore known to play a role in mediating an immune response and inflammation. Identifying such molecules and their role in inflammation provides new targets for treating inflammatory lung diseases, including asthma.

Thus, there exists a need to develop drugs effective in treating inflammatory lung diseases. The present invention satisfies this need and provides related advantages as well.

### SUMMARY OF THE INVENTION

The invention provides an antibody that specifically birds TL1A for use in a method of treating asthma, wherein the antibody blocks TL1A binding to DR3 and decreases the activity of DR3, whereby IL-13 expression is decreased.

### BRIEF DESCRIPTION OF THE DRAWINGS

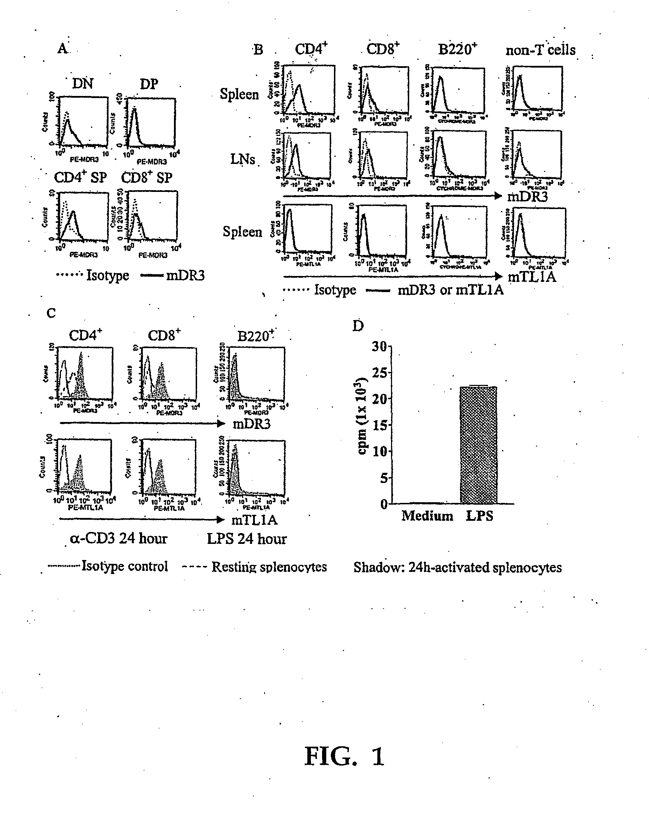

Figure 1. A and B, expression of mDR3 and mTL1A in resting lymphocytes. A. Thymocytes gated on CD4/CD8 double negative (DNA), double positive (DP) or single positive (SP) cells. B. Splenocytes and lymph node cells gated on CD4, CD8, B220 positive cells or CD4, CD8 negative cells. C. Expression of mDR3 and mTL1A on activated cells. Splenocytes were activated with immobilized anti-CD3 (5µg/ml) or LPS (1µg/ml) for 24 hours. D. Proliferation of B cells shows activation of B cells with LPS.

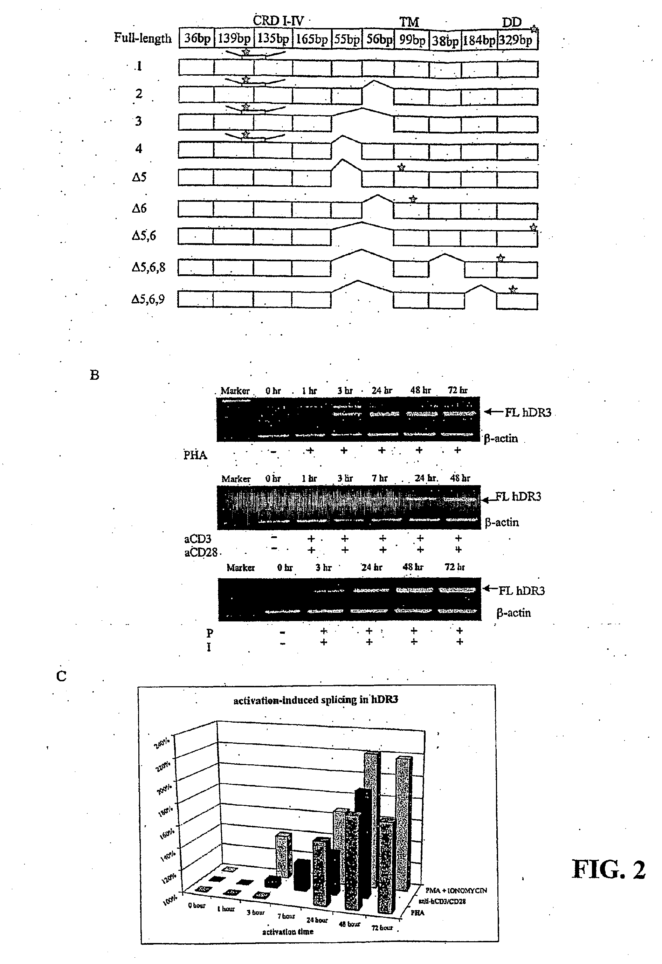

Figure 2. A. Splice forms of mDR3. RT-PCR was performed on mouse cell lines and mouse tissues. RT-PCR products were subcloned into PCR II vector using the TOPO cloning kit, and were confirmed as the splice forms of mDR3 by sequencing. CRD: cysteine-rich domain; TM: transmembrane domain; DD: death domain. Asterisk: stop codon. B. Activation-induced alternative splicing of human DR3. Peripheral blood mononuclear cells (PBMCs) were activated with PHA (5µg/ml), or immobilized anti-hCD3 (5µg/ml) and anti-hCD28 (1µg/ml), or PMA (10ng/ml) and ionomycin (400ng/ml). The cells were harvested at the indicated time points and RT-PCR was performed. Human *β*-actin was used as the internal control. The top band of the three visible bands represents the DR3 splice form retaining the intron between exon 6 and 7; the middle band represents full-length (FL) DR3, and the bottom band shows the splice form lacking exon 6. C. Quantitation of splicing by Molecular Analyst (BioRad, Hercules, CA) software. Ratio was calculated by comparing the intensity of the full-length band with the top band in each sample. Ratio derived from the fresh hPBMCs was designated as 100%.

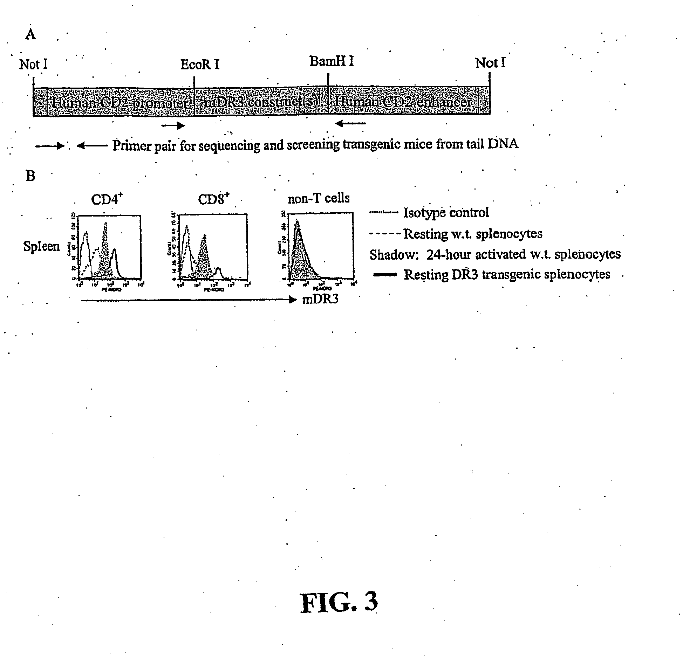

Figure 3. DR3 transgenic mice. A. Mouse DR3 transgenic constructs were under the control of human CD2 promoter and enhancer. B. T cell specific expression of DR3 in transgenic mice in splenocytes gated on CD4 or CD8 positive cells or on CD4/CD8 negative cells.

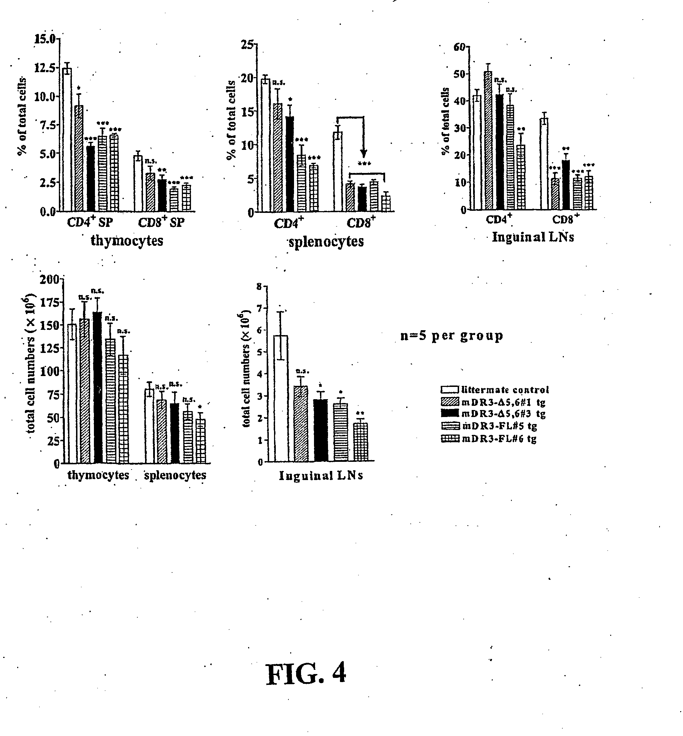

Figure 4. Reduction of CD8 cell number and frequency in mDR3-Δ5,6 and mDR3-FL transgenic mice. Single cell suspensions of thymus, spleen, and inguinal lymph nodes were analyzed. Cells were counted by Trypan Blue exclusion for total cell numbers and stained with CD4-Cyc and CD8-PE for FACS analysis. Statistical calculations were carried out using littermates as controls; n.s.: not significant; * p<0.05; ** p<0.01; *** p<0.001.

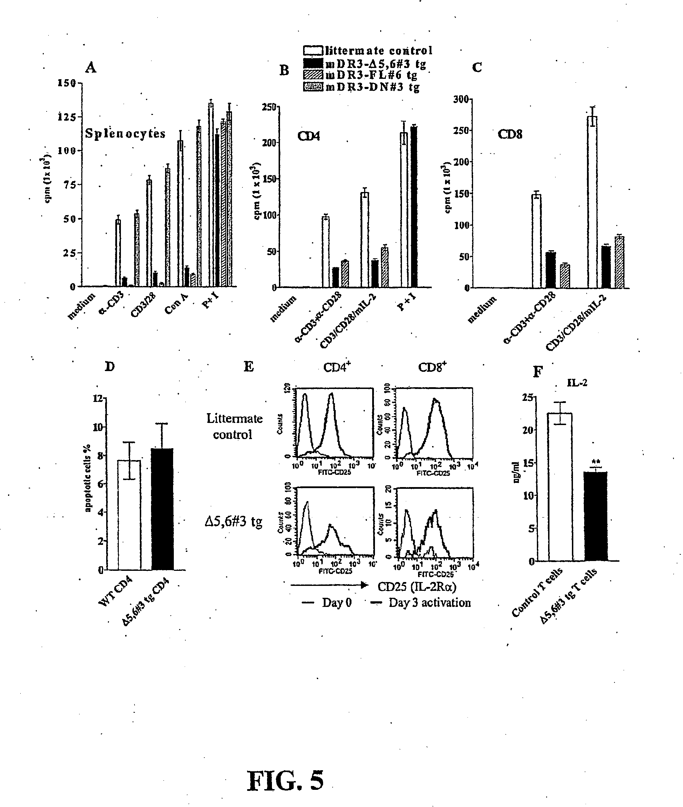

Figure 5. Impaired activation-induced proliferation ofT cells in DR3 transgenic mice. A: Proliferation of splenocytes upon stimulation for 3 days as indicated; B: Proliferation of CD4+ cells or C: CD8+ cells. D: Annexin binding of transgenic and control cells after 72-hour activation with immobilized anti-CD3 and soluble anti-CD28. CD4+ cells were harvested, washed and then stained with Annexin-V-PE and 7-AAD. E: Under the same culture conditions as in D, transgenic and control splenocytes were harvested, washed, and stained with CD25-FITC, CD8-PE, and CD4-CYC. F: Reduced IL-2 production by DR3 transgenic T cells. T cells were purified by negative selection and activated with immobilized anti-CD3 and soluble anti-CD28 for 3 days. Supernatants were assayed for IL-2 production by ELISA assay. ** p 0.0078.

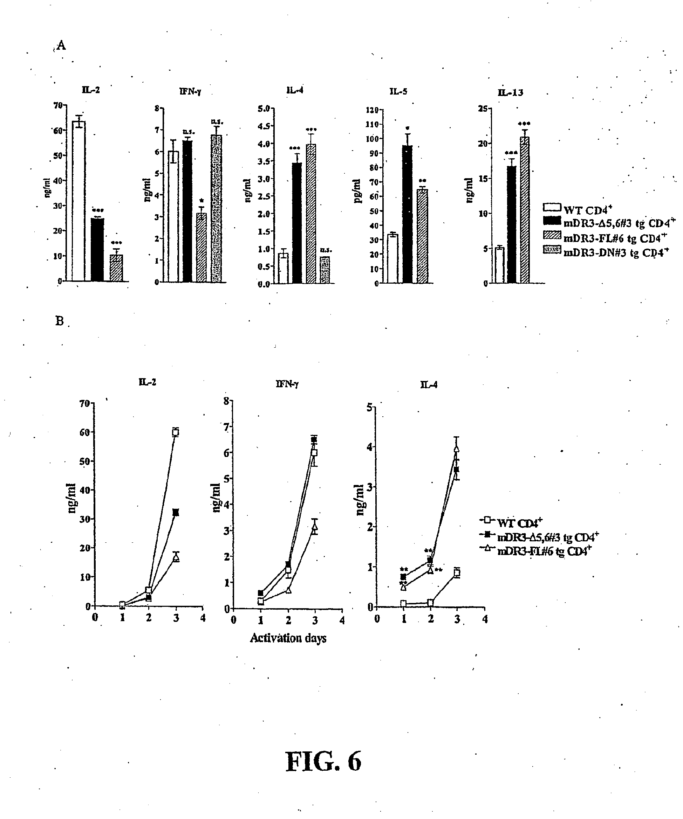

Figure 6. DR3 transgenic CD4+ cells spontaneously produce Th2 cytokines upon activation *in vitro.* A: 1 x 10⁵/well w.t. or DR3 transgenic CD4+ T cells were activated with immobilized anti-CD3 and soluble anti-CD28. Supernatants were collected after 72 hours and cytokine ELISAs were performed. B: CD4+ cells were activated as described in A. Supernatants were collected after 24h, 48h, and 72h culture and analyzed for cytokine production by ELISA. In each experiment, three to four spleens from mDR3-tg were pooled together. Figures represent one of three independent experiments. n.s.: not significant; * p<0.05; ** p<0.01; *** p<0.001

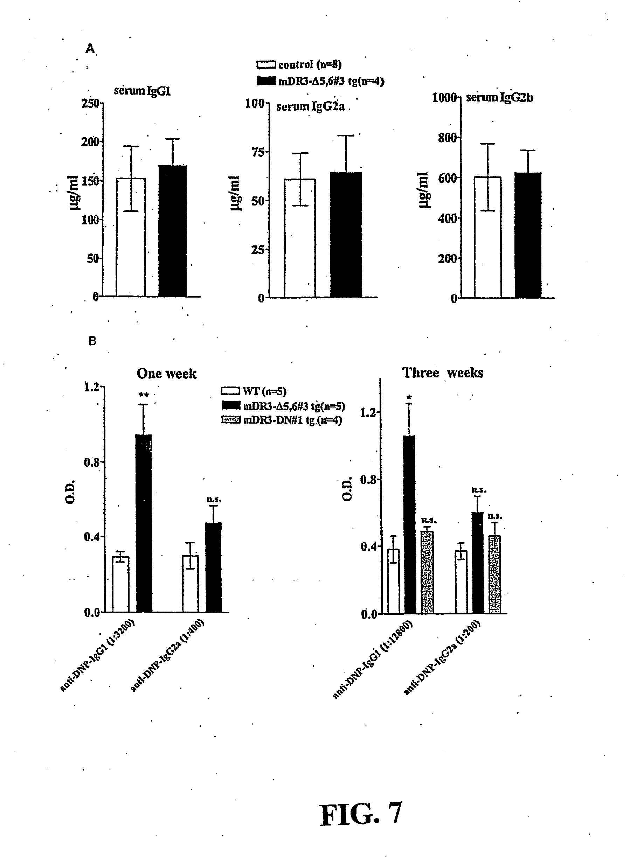

Figure 7. DR3 transgenic mice develop a Th2-biased antibody response after immunization *in vivo.* A: IgG isotypes prior to immunization B: Mice were immunized with 100µg/animal DNP-KLH in sterile PBS and their antigen-specific isotype tested one and three weeks after immunization. High-binding 96-well plates were coated with DNP-BSA at 0.8µg/ml to detect anti-DNP specific antibodies. Figures represent one of three independent experiments. ** p<0.01; * p<0.05; n.s.: not significant:

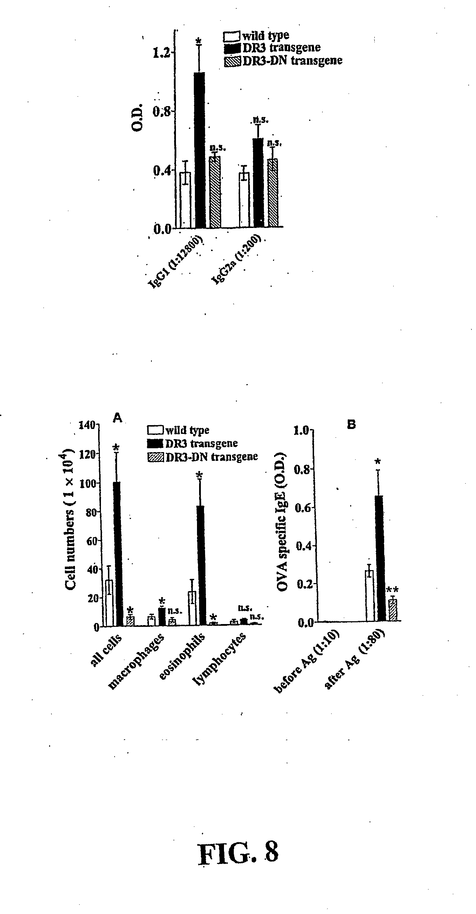

Figure 8. A: Increased lung inflammation in DR3 transgenic mice. Decreased lung inflammation in DR3-DN transgenic mice compared to w.t. Cell numbers and composition in BALF. Animals were sensitized by i.p. injection of 66 µg of ovalbumin and 6.6 mg of alum on days 0 and 5, and challenged with aerosolized 0.5% ovalbumin in PBS on day 12. Differential cell count was done in cytospins of bronchoalveolar lavage cells, stained with Wright-Giemsa. B: Increased specific IgE production in DR3 transgenic and decreased IgE in DR3-DN transgenic mice compared to w.t. mice with experimental asthma. Ovalbumin-specific IgE was detected by ELISA on ovalbumin-coated plates. Five animals per group; n.s.: not significant, *: p<0.05, **: p<0.01 **

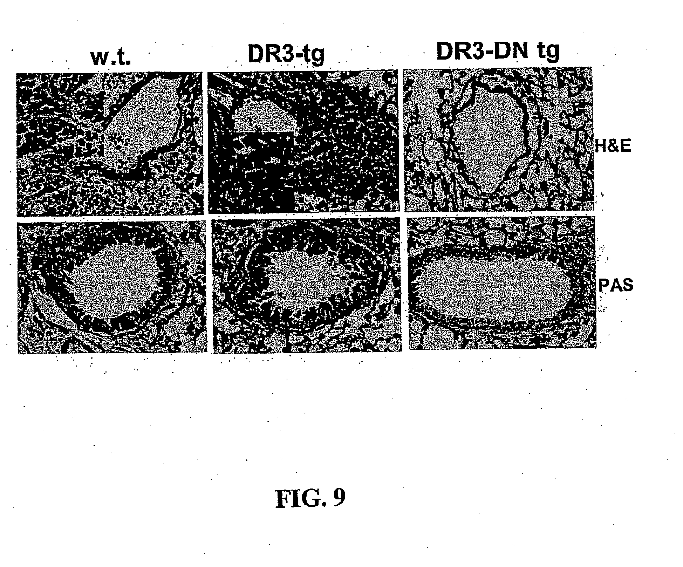

Figure 9. Increased lung inflammation and mucus in DR3-transgenic mice. Decreased inflammation and mucus in DR3-DN transgenic mice. Inflammatory cell infiltration (upper row) and mucus secretion (lower row) in the lungs of w.t., DR3 transgenic mice. The animals were sensitized and challenged as described above. After BALF collection, the lungs were removed, fixed, embedded in paraffin, sectioned and stained with hematoxylin-eosin (upper row) or PAS (lower row). Insets: Higher magnification of infiltrating cells.

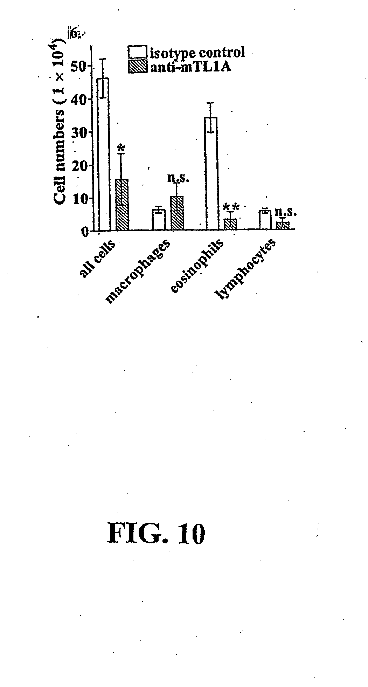

Figure 10. Differential cell counts in BALF of w.t. mice injected with anti-TL1A antibody. Mice were sensitized by i.p. injections of ovalbumin with alum on days 0 and 5, and challenged with aerosolized ovalbumin on day 12. L4G6 antibody or isotype control were injected 50 µg i.p. on days 11, 12, 13 and 14. BALF was collected on day 15, and cell composition was analyzed in cytospins stained with Wright-Giemsa. Three mice per group, n.s.: not significant, *: p<0.05, **: p<0.01.

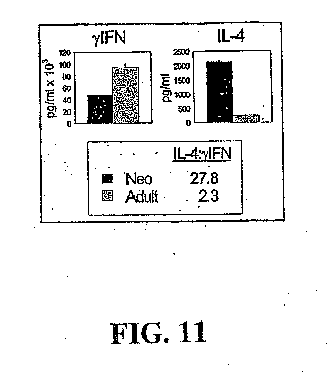

Figure 11. Cytokine responses to KLH immunization in adult and newborn mice. 1 day old newborn and 8 week old adult BALB/c mice were immunized i.p. and s.c. with KLH in PBS; neonates received 10µg and adults 100µg total. Four weeks later, all mice were re-immunized with 100µg KLH in PBS. One week later, CD4+ lymph node cells were prepared and stimulated in the presence of splenic APC with 50µg/ml KLH. Supernatants were harvested after 72 hr of culture and tested for cytokine content using murine-specific ELISA kits. The ratios of IL-4 (pg/ml):IFN-γ (pg/ml x 10³) produced by neonatal vs. adult CD4+ cells are shown. Similar results were obtained using splenic IL-4:IFN-γ CD4+ cells and in the C57BL/6 strain of mouse.

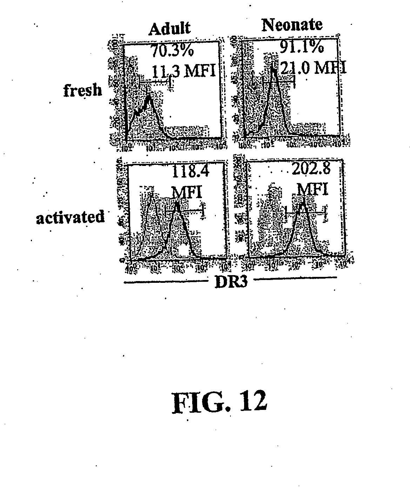

Figure 12. DR3 expression in freshly isolated and Con A-activated CD4+ lymphocytes from adult and newborn mice. Lymph node cells from day 7 or adult BALB/c mice were activated with 2µg/ml Con A and then stained at the indicated times. The staining with the second stage antibody alone (dashed line) or anti-DR3 (solid lines) among gated CD4+ cells is shown. The staining with second stage antibody alone was similar for all time points so just the 22 hr background staining is shown.

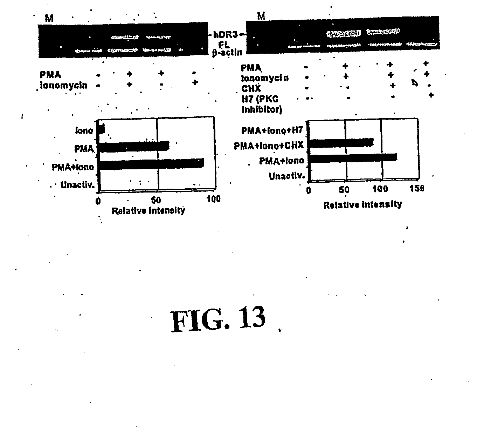

Figure 13. Activation-induced alternative splicing of human DR3. Human PBMCs were activated with PMA (10ng/ml) and ionomycin (400ng/ml) (left) alone and in the presence of inhibitors (right). The cells were harvested after 12 hours and RT-PCR was performed. Human β-actin was used as the internal control. The top band of the three visible bands represents the DR3 splice form retaining the intron between exon 6 and 7; the middle band represents full-length (FL) DR3 (arrow), and the bottom band shows the splice form lacking exon 6. The lower panels show the quantitation of FL-DR3 mRNA expressed as intensity relative to unactivated cells. M - molecular weight marker.

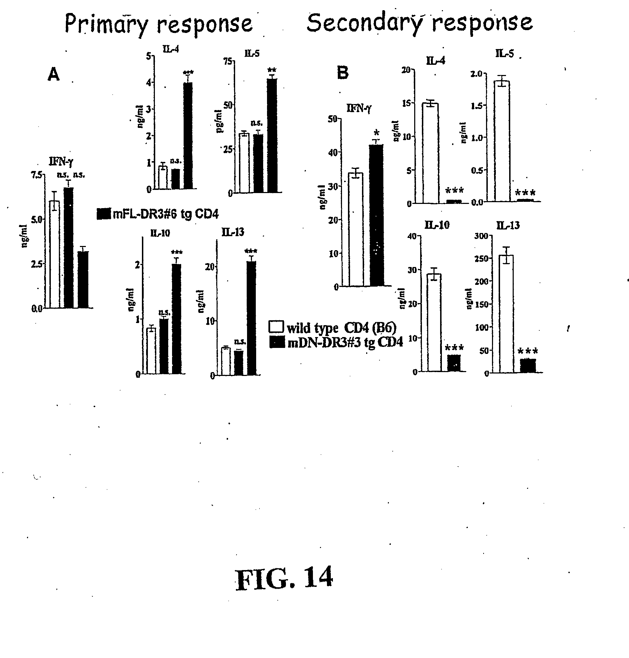

Figure 14. Blockade of DR3 signals by dominant negative DN-DR3 transgenes on T cells blocks Th2 polarization. Transgenic full-length FL-DR3 overexpression on T cells causes increased Th2 cytokine production during primary activation. Purified CD4 cells from w.t., (open bar) FL-DR3 transgenic mice (black) and dominant negative DR3 transgenic (gray) mice were activated for three days with anti-CD3 and anti-CD28. After 72h, supernatants were harvested and analyzed (A). The cells were washed and replated on anti CD3 for additional 48h before analysis of the supernatants (B). Note the different γ-axes in secondary activation and increased production in w.t. CD4 but not DN-DR3 transgenic CD4. n.s. - not significant; * p<0.05; **: p<0.01; ***: p<0.001.

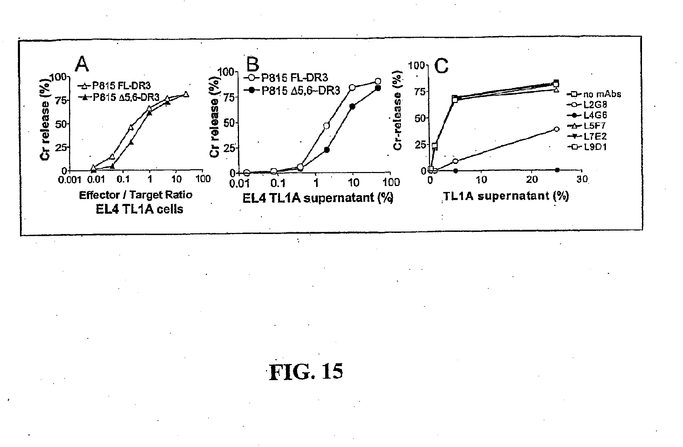

Figure 15. P815 target cells were transfected with FL-mDR3 or with alternatively spliced mΔ5,6-DR3, a form of DR3 lacking exons 5 and 6 encoding part of the extracellular domain. A. EL4 were transfected with mTL1A and used as effector cells at the indicated effector:target ratio with Cr-labeled P815-DR3 or P815-Δ5,6-DR3 in 5 hour assays. B. Supernatants harvested from EL4-TL1A cultures (10⁶/ml, 24h) were used at the indicated concentration with the same P8I5 targets for 5h and Cr release determined. C. Inhibition of TL1A mediated Cr release by monoclonal antibody L4G6, but not by other antibodies. Clone L2G8 shows partial inhibition. Purified L4G6 antibody causes 50% inhibition at 20ng/ml.

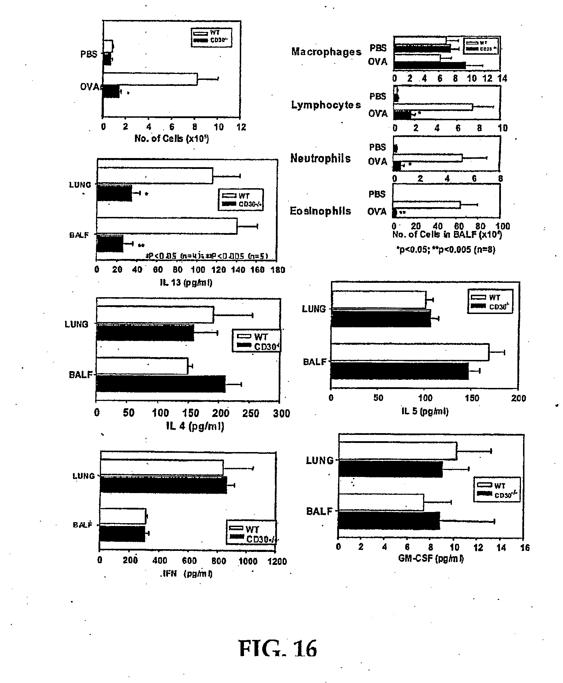

Figure 16 is a series of graphs showing that CD30 deficiency abrogates airway IL-13 production and diminishes cellular exudates in bronchaveolar fluid (BALF) upon antigenic challenge. Mice were immunized i.p. with ovalbumin and alum on day 0 and 5, and challenged with ovalbumin aerosol on day 12. Three days later, BALF was collected by lavage with 3 x 0.5 ml PBS, the lungs were homogenized, and supernatants produced by centrifugation. Cellular exudates in BALF were counted and characterized by Wright Giemsa staining in cytospins. Cytokines were determined by ELISA.

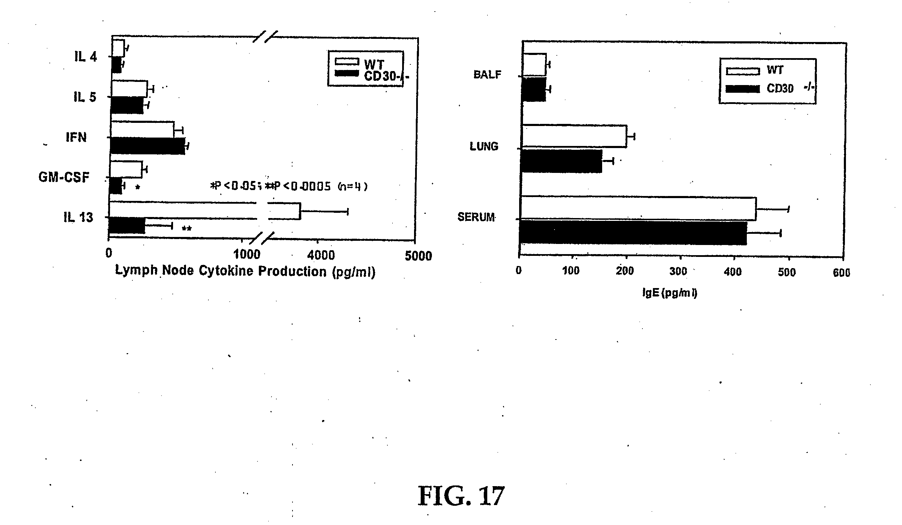

Figure 17 is two graphs showing that CD30 deficiency interferes with IL-13 production in regional lymph nodes and reduces GM-CSF production, but has no influence. on other cytokine levels and on IgE levels in lung or serum. Thoracic lymphocytes isolated from the mice in the experiments described in Figure 1 were re-stimulated *in vitro* with 1 mg/ml ovalbumin for three days. IgE levels in the lung and in serum were measured by ELISA.

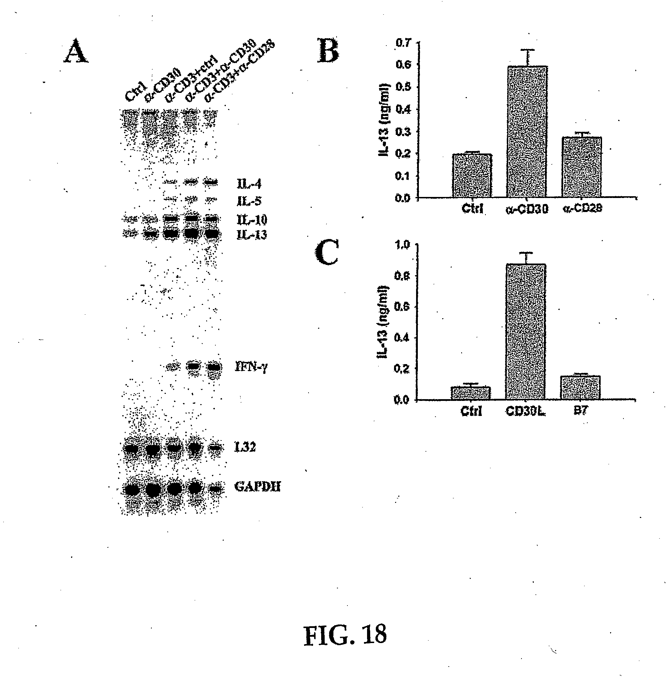

Figure 18 is a blot and graphs showing that CD30 signals for IL-13 production are TCR independent. DO11 TCR transgenic T cells were activated with ovalbumin for 5 days to induce CD30 expression and, after washing, restimulated with anti-CD30 antibody or CD30-L (CD30-Ligand) transfected P815 cells, in the absence or presence of anti-CD3 and anti-CD28 antibodies or P815-B7 (B7 transfected P815 cells) as indicated. Cytokine mRNA levels were determined by RNA protection assays (A); IL-13 production was measured by ELISA (B, C).

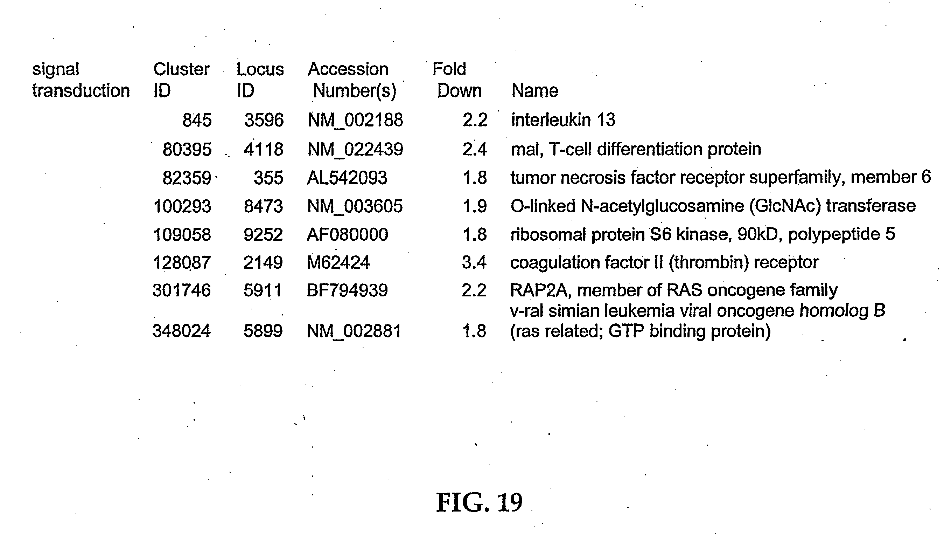

Figure 19 is a table listing genes up-regulated in response to CD30 signaling.

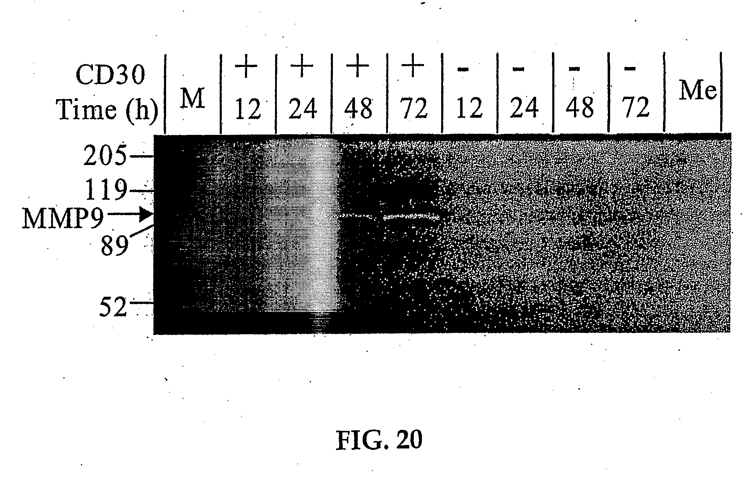

Figure 20 is a photograph of a gel showing that matrix metalloproteinase (MMP9) secretion is induced by CD30 signals. CD30 positive YT cells (LGL lymphoma, 2 x 10⁵/ml) were treated with 5µg/ml agonistic anti-CD30 antibody (C10) for the periods indicated in serum-free aim 5 medium and supernatants harvested. Controls received no antibody. Me: medium only. The supernatants were analyzed by zymography on SDS-PAGE, incorporating 0.33mg/ml gelatin in the gel matrix. MMP9 digests gelatin to leave a gelatin-free band appearing white after staining with Coomassie blue. M - Markers in kD.

Figures 2 1 A and 21 B show the nucleotide (SEQ ID NO:1) and amino acid (SEQ ID NO:2) sequences, respectively, of human DR3 (GenBank accession No. X63679).

Figures 22A and 22B show the nucleotide (SEQ ID NO:3) and amino acid (SEQ ID NO:4) sequences, respectively, of human CD30 (GenBank accession No. M83554).

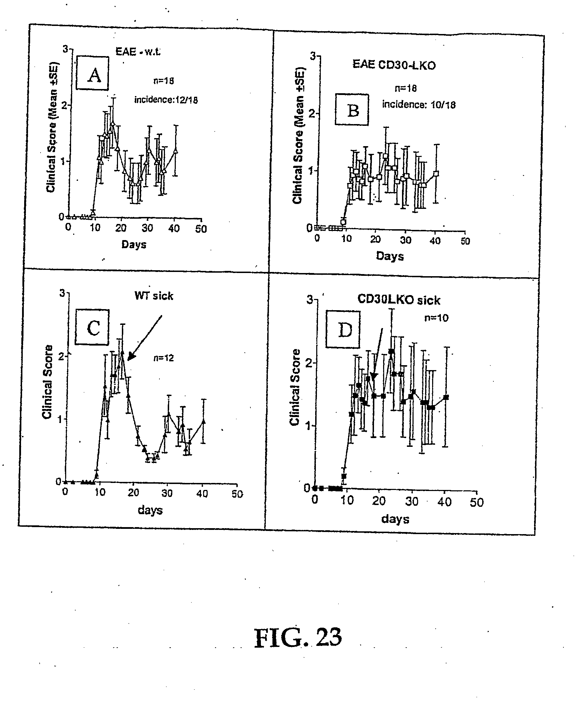

Figure 23 is a series of graphs showing that EAE does not resolve in CD30-Ligand knock out (k.o.) mice. Wild type and CD30-Ligand knock out mice (CD30-LKO) were injected on day 0 with MOG (a major oligodendrocyte glycoprotein-derived peptide) under conditions known to induce EAE. The clinical score of disease is shown: 0 no disease, 6 dead; 1-5 increasing, ascending paralysis beginning at the tail. A: score of all 18 mice injected, including in the mean mice that did not get sick; disease incidence 12/18. C: same data as in A, but only plotting the score of mice that became sick. B and C, identical procedure and analysis in CD30-LKO. Arrows in C and D point to spontaneous resolution in w.t. but not in CD30-LKO.

Figure 24 is a series of graphs showing that anti-CD30 interferes with resolution of disease in w.t. mice (upper panels) and aggravates EAE in CD30-LKO (lower panels). Mice were injected with MOG as in Figure 23. On day 0, 4, 7 and 12, the mice also received 100 µg anti-CD30 antibody intraperitoneally.

Figure 25 shows expression of mDR3 in lymph nodes of B6 wt mice and DR3 transgenic mice measured by flow cytometry.

Figure 26 shows expression of mTL1A in bronchial lymph nodes (LNs) of ovalbumin sensitized and aerosol challenged B6 wt mice. Figure 26A shows that anti-mTL1A monoclonal antibody stained mTL1A on TL1A-transfected P815 cells, but not untransfected cells. Figure 26B shows that expression of mTL1A was only detected on a portion of CD11c expressing dendritic cells (DCs) (arrow) in bronchial lymph node cells from ovalbumin (OVA) sensitized and aerosol challenged B6 wt mice.

### DETAILED DESCRIPTION OF THE INVENTION

The invention provides antibodies for use in methods for treating asthma. The antibodies decrease the activity of DR3 DR3 is a member of the tumor necrosis factor receptor (TNFR) family. As disclosed herein, decreasing the activity or expression of DR3 decreases the expression of interleukin-13 (IL-13). Decreasing the expression of IL-13 can be used to ameliorate a sign or symptom associated with an inflammatory disease. The antibodies are useful for treating inflammatory lung disease, asthma.

In one embodiment, the invention is based on the further characterization of the physiological function of DR3 on peripheral T cells and the discovery that DR3 plays an important role in the development of inflammatory lung disease (asthma). In making the invention, DR3 transgenic mice expressing DR3 under the T cell-specific CD2 promoter were created. In order to gain insight into the biological function of alternatively spliced versions of DR3, murine transgenes were generated for full-length DR3 (DR3-FL), for an alternatively spliced but membrane-associated version of DR3 (DR3-Δ5,6), and for a dominant negative version of DR3 (DR3-DN) lacking the intracellular domain. The data obtained indicated that DR3 is upregulated very early during T cell activation by alternative splicing and that it contributes to the regulation of Th1/Th2 polarization of CD4 cells.

The full-length DR3 transgene supported the production of Th2 cytokines (IL-4, IL-5, IL-13 and IL-10) and suppressed IFN-γ secretion during primary activation of CD4 cells. In contrast, the dominant negative DR transgene that blocked DR3 signaling had no effect on cytokine production during primary activation. The lack of an effect of DN-DR3 transgenes during primary activation suggests that DR3 signals are not produced in wild-type (w.t.) cells during priming. Secondary activation of w.t. CD4 cells with anti-CD3 results in a 5-10 fold increased production of both Th1 and Th2 cytokines. In contrast, the presence of the DN-DR3 transgene completely and selectively blocked the increased production of Th2 cytokines (including IL-10) but left IFN-γ production unaffected.

The physiological relevance of these findings was demonstrated in animal experiments that showed that DR3 is a critical receptor in the induction of inflammatory lung disease. DR3 transgenic mice expressing DR3 under the T cell-specific CD2 promoter were created. In order to gain insight into the biological function of alternatively spliced versions of DR3, murine transgenes were generated for full-length DR3 (DR3-FL), for an alternatively spliced but membrane-associated version of DR3 (DR3-Δ5,6), and for a dominant negative version of DR3 (DR3-DN) lacking the intracellular domain. The data obtained indicated that DR3 is upregulated very early during T cell activation by alternative splicing and that it contributes to the regulation of Th1/Th2 polarization of CD4 cells. In particular, DR3 transgenic mice expressing full-length DR3 on T cells and dominant negative DR3-DN transgenic mice in which DR3 signals on T cells are blocked by the dominant negative form of DR3 were created and used in a murine ovalbumin asthma model. In the model, DR3 transgenic mice displayed exaggerated lung inflammation compared to non-transgenic litter mates. In contrast, mice expressing the dominant negative form of DR3 displayed no lung inflammation.

These results were validated using an antibody that binds to and blocks TL1A (TNFSF15) engagement of DR3. Administration of anti-TL1A antibodies to normal ovalbumin primed mice during airway ovalbumin challenge in the murine ovalbumin asthma model completely blocked the inflammatory lung response seen without antibody treatment or with control (non-TL1A-specific) antibody. Because newborn mice and children exhibit a striking Th2 bias that upon normal development of the immune system transitions to a Th1 bias in adults, expression of DR3 on T cells in newborn mice was compared to that in adult mice. DR3 was expressed at higher levels in the newborn mice, suggesting that its expression is correlated with the Th2 bias of developing mice.

Unlike that of any other member of the TNF-R family, DR3 expression was found to be controlled by alternative mRNA splicing. Resting T cells expressed little or no DR3 protein, but contained high levels of randomly spliced DR3 mRNA. Upon T cell activation via the T cell receptor, protein kinase C (PKC) was activated. PKC activation in turn mediated correct splicing of full-length DR3 and surface expression of the protein. This unique regulation of DR3 expression allows for rapid DR3 protein expression on T cells and enables environmental regulation of DR3 expression via influencing PKC levels responsible for DR3 splicing and expression.

There is described the discovery that CD30 plays an important role in regulating T lymphocyte responses involved in the pathogenesis of asthma. In particular, it was shown (i) that signaling through CD30 can trigger IL-13 production even in the absence of T cell receptor stimulation, and (ii) that, compared to control mice, CD30 and CD30-Ligand knockout mice exhibit diminished eosinophilia and IL-13 production after airway challenge in a murine model of AHR. Thus, modulating the function of CD30 should lead to decreased IL-13 production and reduced airway inflammation.

There is described the discovery that CD30 plays an important role in regulating T lymphocyte responses involved in normal and aberrant immune system responses. In particular, using mice with experimental autoimmune encephalitis (EAE; a model for human multiple sclerosis), it was shown that CD30-mediated signals play an important role in the resolution of the disease. In other studies, stimulation of CD30 (i) induced T cells to produce IL-13 production and (ii) up-regulated CCR7, a homing receptor that directs T cells to lymph nodes. In the absence of CD30 signaling, T cells do not produce IL-13 and do not traffic back to lymph nodes.

There are described methods for both up-regulating a T cell-mediated immune response (for example, to upregulate an anti-tumor response by enhancing CD30 signals) and for suppressing the down-regulation of an immune response (for example, to maintain immune responses against tumors). Conversely, there are described methods for diminishing the immune response by blocking CD30 signals and for enhancing the down regulation of an ongoing immune response by enhancing CD30 signals.

Accordingly there is described a method of treating a reactive airway disease in an animal subject. The method includes the step of administering to the subject an agent which modulates at least one functional activity of CD30. There is also described a method of modulating a T cell response in an animal subject. The method includes the step of administering to the subject an agent which modulates at least one functional activity of CD30.

The agent used in the method can be an antibody, for example, one that specifically binds CD30 or CD30-ligand. It can also be CD30, CD30-Ligand, or a derivative or variant thereof such as a CD30-immunoglobulin fusion protein.

The agent which modulates at least one functional activity of CD30 can also take the form of a nucleic acid, for example, an antisense construct, a ribozyme, or a RNAi construct. It can also be one that causes a gene encoding CD30 or CD30-Ligand to become non-functional or one that interferes with transmembrane signaling mediated by CD30, for example, an agent that targets TRAF2 or p38.

Unless otherwise defined, all technical terms used herein have the same meaning as commonly understood by one of ordinary skill in the art to which this invention belongs. Commonly understood definitions of molecular biology terms can be found in Rieger et al., Glossary of Genetics: Classical and Molecular, 5th edition, Springer-Verlag: New York, 1991; and Lewin, Genes V, Oxford University Press: New York, 1994.

As used herein, "protein" or "polypeptide" means any peptide-linked chain of amino acids, regardless of length or post-translational modification, for example, glycosylation or phosphorylation.

By the term "ligand" is meant a molecule that will bind to a complementary site on a given structure. For example, a CD30 ligand (CD30-Ligand) binds CD30, and TL1A is a ligand for DR3.

The term "specifically binds", as used herein, when referring to a polypeptide, including antibodies, or receptor, refers to a binding reaction which is determinative of the presence of the protein or polypeptide or receptor in a heterogeneous population of proteins and other biologics. Thus, under designated conditions, for example, immunoassay conditions in the case of an antibody, the specified ligand or antibody binds to its particular "target" (for example, a CD30 ligand specifically binds to CD30) and does not bind in a significant amount to other proteins present in the sample or to other proteins to which the ligand or antibody may come in contact in an organism. Generally, a first molecule that "specifically binds" a second molecule has a binding affinity greater than about 10⁵, for example, 10⁶,10⁷, 10⁸, 10⁹, 10¹⁰, 10¹¹, and 10¹² or more, moles/liter.

A "functional activity of CD30" is any activity normally performed by CD30. For example, functional activities include the ability to specifically bind CD30-Ligand, the ability to cause transmembrane signaling through TRAF2 and P38, and the ability to cause increased IL-13 production by T lymphocytes when stimulated.

As used herein, the term "inflammatory lung disease" refers to a disease associate with an inflammatory or immune response in the lung. Exemplary inflammatory lung diseases include, for example, asthma, acute lung injury, adult respiratory distress syndrome, emphysema, chronic bronchitis, cystic fibrosis, and interstitial lung disease such as interstitial pneumonitis, idiopathic fibrosis and interstitial fibrosis.

Asthma is a disease of localized anaphylaxis, or atopy, and is characterized as an inflammatory disease. In some cases, asthma is triggered by exposure to allergens (allergic asthma), while in other cases, asthma is triggered independent of allergen stimulation (intrinsic asthma). Upon inhalation of an allergen by an asthmatic individual, an immune response is initiated, resulting in the release of mediators of hypersensitivity including histamine, bradykinin, leukotrienes, prostaglandins, thromboxane A2 and platelet activating factor. The initial phase of the asthmatic response also results in the release of chemotactic factors that recruit inflammatory cells such as eosinophils and neutrophils. Clinical manifestations of these events include occlusion of the bronchial lumen with mucus, proteins and cellular debris; sloughing of the epithelium; thickening of the basement membrane; fluid buildup (edema); and hypertrophy of the bronchial smooth muscles.

Acute lung injury occurs when an insult to the lung causes an acute inflammatory reaction, which results in respiratory distress, hypoxemia and diffuse alveolar infiltrates and can lead to respiratory failure. Acute lung injury can occur with a variety of pulmonary insults, including, for example, sepsis and trauma. The extent of acute lung injury depends, for example, on the magnitude of initial damage, repeated insults such as persistent septicemia or retained necrotic and inflamed tissue, and added insults from treatment including barotrauma, hyperoxia and nosocomial infection.

Adult respiratory distress syndrome (ARDS) is a form of acute lung injury often seen in previously healthy patients. ARDS is characterized by rapid respiratory rates, a sensation of profound shortness of breath, sever hypoxemia not responsive to supplemental oxygen, and widespread pulmonary infiltrates not explained by cardiovascular disease or volume overload. ARDS tends to follow a diverse array of systemic and pulmonary insults, although the majority of ARDS is associated with systemic or pulmonary infection, severe trauma, or aspirating gastric contents. The crucial stimulus to the development of ARDS is an inflammatory response to distant or local tissue injury. Disorders associated with ARDS include aspiration of gastric contents, fresh and salt water and hydrocarbons; central nervous system trauma, anoxia, seizures or increased intracranial pressure;,drug overdose or reactions; hematologic alterations; infection including sepsis, pneumonia and tuberculosis; inhalation of toxins such as oxygen, smoke or corrosive chemicals; metabolic disorders such as pancreatitis; shock; and trauma such as fat emboli, lung contusion, severe nonthoracic trauma and cardiopulmonary bypass.

Interstitial lung disease includes, for example, idiopathic fibrosis, interstitial fibrosis and interstitial pneumonitis. Interstitial pneumonitis, also known as hypersensitivity pneumonitis, results from inhaling diverse environmental antigens and chemicals. Symptoms of the disease include wheezing and dyspnea, and the disease is associated with infiltration of alveolar walls with lymphocytes, plasma cells, and other inflammatory cells. The disease can be an acute illness or can be present in a chronic form with pulmonary fibrosis upon progression to interstitial fibrotic disease with restrictive pattern on pulmonary function.

Chronic bronchitis is an inflammation of the bronchial tubes and can generally be manifested in two forms. "Simple chronic bronchitis" is correlated to exposure to environmental irritants, including occupational exposure to dust, grains and mining as well as cigarette smoking. Exposure to such environmental irritants is associated with inflammatory changes in the airways.

Another form of chronic bronchitis is "chronic obstructive bronchitis," which is also strongly correlated with cigarette smoking. Patients exhibiting chronic obstructive bronchitis often have emphysema, which is similarly associated with cigarette smoking. Emphysema is associated with the chronic, progressive destruction of the alveolar structure and enlarged air spaces. The destruction of the alveolar structure is associated with proteases released by neutrophils (polymorphonuclear leukocyte; PMN) recruited into the lung by pulmonary alveolar macrophages. Symptoms of emphysema include undue breathlessness upon exertion.

Cystic fibrosis is a lethal genetic disease characterized by abnormally viscous mucous secretions, which lead to chronic pulmonary disease. Defective chloride ion secretion occurs in cystic fibrosis due to mutations in an epithelial cell chloride ion channel, the cystic fibrosis transmembrane regulator (CFTR). Disease progression is often marked by gradual decline in pulmonary function. The major source of morbidity in cystic fibrosis patients is pulmonary disease associated with chronic and recurrent bacterial infections and the detrimental cumulative long-term effects of the resulting inflammatory response on the pulmonary tissue.

As used herein, the term "treating an inflammatory lung disease" refers to the amelioration of a sign or symptom associated with the inflammatory lung disease. Treating an inflammatory lung disease is intended to encompass a reduction in the onset or magnitude of a sign or symptom associated with an inflammatory lung disease. One skilled in the art can readily recognize and determine the amelioration of a sign or symptom associated with a particular inflammatory lung disease.

In one embodiment, the disclosure relates to a method of modulating a T cell immune response. The method includes the step of modulating DR3 function in the T cell, wherein the T cell response causes a symptom of inflammatory lung disease. T cell located within an animal subject comprises contacting the cell with an agent that blocks the interaction of DR3 and TL1A. The agent is an antibody that specifically binds TL1A.

The disclosure additionally relates to a method of modulating a T cell immune response by modulating DR3 function in the T cell. Such a method is useful for modulating a T cell located within an animal subject.

There is described a method of treating a reactive airway disease in an animal subject The method can include the step of administering to the subject an agent which modulates at least one functional activity of CD30. The agent can be an antibody, for example, an antibody that specifically binds CD30 or CD30-ligand. The agent can be CD30 or CD30-Ligand. The agent can be a CD30-immunoglobulin fusion protein. The agent can be a nucleic acid, for example, an antisense construct, a ribozyme, or a RNAi construct. The agent can be one that causes a gene encoding CD30 or CD30-Ligand to become non-functional. The agent can be one that interferes with transmembrane signaling mediated by CD30. The agent can target TRAF2 or p38.

There is described a method of modulating T cell responses in an animal subject. The method can include the step of administering to the subject an agent which modulates at least one functional activity of CD30. Similar agents as those described above for modulating CD30 activity for treating reactive airway disease or inflammatory lung diseases, as disclosed herein, can be used in such a method. In addition, the agent can be a CD30-Ligand-CD8-fusion protein to modulate a functional activity of CD30.

The disclosure additionally relates to a method for treating an inflammatory lung disease by administering an agent that decreases the activity of DR3 whereby IL-13 expression is decreased. The inflammatory lung disease is asthma. The agent decreases activity of DR3 . The agent is an antibody that binds a DR3 ligand TL1A.

There is described that an agent can decrease expression of DR3 or CD30. The agent can be, for example, a nucleic acid. The nucleic acid can encode an antisense nucleic acid, a ribozyme or an RNA interference construct. A composition can be administered containing one or more agents that decrease the activity of DR3 and CD30.

There is described an antibody for modulating a T cell response or reactive airway disease in an animal subject by administering to the subject an agent which modulates at least one functional activity of DR3

### Biological Methods

The methods and compositions described herein utilize conventional techniques in the biological sciences. Such teclmiques are generally known in the art and are described in detail in methodology treatises such as Molecular Cloning: A Laboratory Manual, 3rd ed., vol. 1-3, ed. Sambrook et al., Cold Spring Harbor Laboratory Press, Cold Spring Harbor, N.Y., 2001; and Current Protocols in Molecular Biology, ed. Ausubel et al., Greene Publishing and Wiley-Interscience, New York, 1992 (with periodic updates). Immunological methods, for example, preparation of antigen-specific antibodies, immunoprecipitation, and immunoblotting, are described, for example, in Current Protocols in Immunology, ed. Coligan et al., John Wiley & Sons, New York, 1991; Methods of Immunological Analysis, ed. Masseyeff et al., John Wiley & Sons, New York, 1992; and Harlow and Lane, Antibodies: A Laboratory Manual (Cold Spring Harbor Laboratory Press (1988). Conventional methods of gene transfer and gene therapy can also be adapted for use in the present invention, as described, for example, in Gene Therapy: Principles and Applications, ed. T. Blackenstein, Springer Verlag, 1999; Gene Therapy Protocols (Methods in Molecular Medicine), ed. P.D. Robbins, Humana Press, 1997; and Retro-vectors for Human Gene Therapy, ed. C.P. Hodgson, Springer Verlag, 1996.

### CD30

There is described modulation of CD30 as a treatment or prophylactic for inflammatory lung disease, including airway hyper-reactivity (AHR), particularly asthma. There is also described modulation of CD30 as a method for regulating T cell responses. CD30 is a 595 amino acid protein originally described by Durkop et al. (Cell 68:421, 1992). It is a member of the TNF receptor superfamily, has five cysteine-rich repeats, and is expressed on mitogen-activated B and T cells. CD30 binds its ligand CD30L (also known as CD153) to co-stimulate T-cell activation. CD30L is a CD30-binding protein originally described by Smith et al. (Cell 73:1349, 1993). It is expressed on T and B cells, monocytes/macrophages, neutrophils, megakaryocytes, erythroid precursors and eosinophils.

### Modulating a DR3 or CD30 Functional Activity

Methods of the disclosure utilize an agent that modulates at least one functional activity of DR3

Agents that bind DR3 are antibodies and antibody fragments that specifically bind TL1A Similarly, agents that bind TL1A are antibodies and antibody fragments that specifically bind TL1A and muteins thereof. anti-TL1A antibodies can be made according to known methods such as those described herein.

Antibodies used in methods of the disclosure include polyclonal antibodies and, in addition, monoclonal antibodies, single chain antibodies, Fab fragments, F(ab')₂ fragments, and molecules produced using a Fab expression library. Monoclonal antibodies, which are homogeneous populations of antibodies to a particular antigen, can be prepared using the DR3-Ligand (TL1A) protein described above and standard hybridoma technology (see, for example, Kohler et al., Nature 256:495, 1975; Kohler et al., Eur. J. Immunol. 6:511, 1976; Kohler et al., Eur. J. Immunol. 6:292, 1976; Hammerling et al., "Monoclonal Antibodies and T Cell Hybridomas," Elsevier, N.Y., 1981; Ausubel et al., supra). In particular, monoclonal antibodies can be obtained by any technique that provides for the production of antibody molecules by continuous cell lines in culture such as described in Kohler et al., Nature 256:495, 1975, and U.S. Pat. No. 4,376,110; the human B-cell hybridoma technique (Kosbor et al., Immunology Today 4:72, 1983; Cole et al., Proc. Natl. Acad. Sci. USA 80:2026, 1983), and the EBV-hybridoma technique (Cole et al., "Monoclonal Antibodies and Cancer Therapy," Alan R. Liss, Inc., pp. 77-96, 1983). Such antibodies can be of any immunoglobulin class including IgG, IgM, IgE, IgA, IgD and any subclass thereof.

The antibodies of the disclosure thus include naturally occurring antibodies as well as non-naturally occurring antibodies, including, for example, single chain antibodies, chimeric, bifunctional and humanized antibodies, as well as antigen-binding fragments thereof. Such non-naturally occurring antibodies can be constructed using solid phase peptide synthesis, can be produced recombinantly or can be obtained, for example, by screening combinatorial libraries consisting of variable heavy chains and variable light chains as described by Huse et al. (Science 246:1275-1281 (1989)). These and other methods of making functional antibodies are well known to those skilled in the art (Winter and Harris, Immunol. Today 14:243-246 (1993); Ward et al., Nature 341:544-546 (1989); Harlow and Lane, supra, 1988); Hilyard et al., Protein Engineering: A practical approach (IRL Press 1992); Borrabeck, Antibody Engineering, 2d ed. (Oxford University Press 1995)).

To modulate a DR3 the antibody can sterically hinder the physical interaction of DR3 and TL1A An antibody to TL1A can modulate a DR3 by hindering the interaction of DR3 and TL1A

In addition to antibodies, naturally occurring and engineered agents that specifically bind DR3, TL1A, CD30 or CD30 ligand are described. Agents that specifically bind DR3 or CD30 can act as agonists or antagonists. Agents that specifically bind AL1A or CD30 ligand can obstruct DR3-TL1A or CD30-CD30 ligand interactions, respectively. An example of a naturally occurring agent that specifically binds TL1A is a soluble form of DR3. Similarly, a naturally occurring agent that specifically binds CD30 ligand is a soluble form of CD30. An example of an engineered agent that specifically binds TL1A is a DR3-immunoglobulin fusion protein, and an example of an engineered agent that specifically binds CD30 ligand is a CD30-iunmunoglobulin fusion protein.

Agents that down-regulate expression of DR3, TL1A, CD30, or CD30 ligand are also useful. In the instance of DR3, the protein is expressed on the membrane only after correct splicing of preexisting, but randomly spliced mRNA. Correct splicing was shown to be mediated by PKC activation. Therefore inhibitors of PKC or down stream signaling intermediates will be efficient inhibitors of DR3 signals.

A number of different agents might be employed for this purpose including ribozymes, and antisense and RNA interference (RNAi) constructs. Useful antisense nucleic acid molecules are those that specifically hybridize under cellular conditions to cellular mRNA and/or genomic DNA encoding DR3, TL1A, CD30 or CD30 ligand in a manner that inhibits expression of the protein, for example, by inhibiting transcription and/or translation. The binding may be by conventional base pair complementarity, or, for example, in the case of binding to DNA duplexes, through specific interactions in the major groove of the double helix.

Antisense constructs can be delivered, for example, as an expression plasmid which, when transcribed in the cell, produces RNA which is complementary to at least a unique portion of the cellular mRNA which encodes DR3, TL1A, CD30 or CD30 ligand. Alternatively, the antisense construct can take the form of an oligonucleotide probe generated *ex vivo* which, when introduced into a DR3, TL1A, CD30 or CD30 ligand-expressing cell, causes inhibition of protein expression by hybridizing with an mRNA and/or genomic sequences coding for DR3, TL1A, CD30 or CD30 ligand. Such oligonucleotide probes are preferably modified oligonucleotides that are resistant to endogenous nucleases, for example, exonucleases and/or endonucleases, and are therefore stable *in vivo.* Exemplary nucleic acid molecules for use as antisense oligonucleotides are phosphoramidate, phosphothioate and methylphosphonate analogs of DNA (see, for example, U.S. Pat. Nos. 5,176,996; 5,264,564; and 5,256,775). Additionally, general approaches to constructing oligomers useful in antisense therapy have been reviewed (see, for example, Van der Krol et al. (1988) Biotechniques 6:958-976; and Stein et al. (1988) Cancer Res 48:2659-2668. Methods for selecting and preparing antisense nucleic acid molecules are well known in the art and include in silico approaches (Patzel et al. Nucl. Acids Res. 27:4328-4334 (1999); Cheng et al., Proc. Natl. Acad. Sci., USA 93:8502-8507 (1996); Lebedeva and Stein, Ann. Rev. Pharmacol. Toxicol. 41:403-419 (2001); Juliano and Yoo, Curr. Opin. Mol. Ther. 2:297-303 (2000); and Cho-Chung, Pharmacol. Ther. 82:437-449 (1999); Mir and Southern, Nature Biotech. 17:788-792 (1999)). With respect to antisense DNA, oligodeoxyribonucleotides derived from the translation initiation site, for example, between the -10 and +10 regions of a DR3, TL1A, CD30 or CD30 ligand encoding nucleotide sequence, are preferred.

A number of methods have been developed for delivering antisense DNA or RNA into cells. For instance, antisense molecules can be introduced directly into a cell by electroporation, liposome-mediated transfection, CaPO₄-mediated transfection, viral vector infection, or using a gene gun. Modified nucleic acid molecules designed to target the desired cells, for example, antisense oligonucleotides linked to peptides or antibodies that specifically bind receptors or antigens expressed on the target cell surface, can be used. To achieve high intracellular concentrations of antisense oligonucleotides, as may be required to suppress translation on endogenous mRNAs, a preferred approach utilizes a recombinant DNA construct in which the antisense oligonucleotide is placed under the control of a strong promoter, for example, the CMV promoter.

Ribozyme molecules designed to catalytically cleave DR3, TL1A, CD30 or CD30 ligand mRNA transcripts can also be used to prevent translation of DR3, TL1A, CD30 or CD30 ligand mRNAs and expression of DR3, TL1A, CD30 or CD30 ligand proteins (see, for example, Wright and Kearney, Cancer Invest. 19:495, 2001; Lewin and Hauswirth, Trends Mol. Med. 7:221,2001; Sarver et al. Science 247:1222-1225, 1990; Hauswirth and Lewin, Prog. Retin. Eye Res. 19:689-710 (2000); Ke et al., Int. J. Oncol. 12:1391-1396 (1998); Doherty et al., Ann. Rev. Biophys. Biomol. Struct. 30:457-475 (2001); Bartel and Szostak, Science 261:1411-1418 (1993); Breaker, Chem. Rev. 97:371-390 (1997); and Santoro and Joyce, Proc. Natl. Acad. Sci., USA 94:4262-4266 (1997); and U.S. Pat. No. 5,093,246). As one example, hammerhead ribozymes that cleave mRNAs at locations dictated by flanking regions that form complementary base pairs with the target mRNA might be used so long as the target mRNA has the following common sequence: 5'-UG-3' (see, for example, Haseloff and Gerlach Nature 334:585-591, 1988). To increase efficiency and minimize the intracellular accumulation of non-functional mRNA transcripts, a ribozyme should be engineered so that the cleavage recognition site is located near the 5' end of the target mRNA. Ribozymes can be delivered to a cell using a vector

Where a ribozyme is to be administered to a subject without being delivered using a viral or other vector, the ribozyme can be modified, if desired, to enhance stability. Modifications useful in a therapeutic ribozyme include, but are not limited to, blocking the 3' end of the molecule and the 2' positions of pyrimidines. Stabilized ribozymes can have half-lives of hours and can be administered repeatedly using, for example, intravenous or topical injection. Those skilled in the art understand that a ribozyme also can be administered by expression in a viral gene therapy vector.

Other methods can also be used to reduce DR3, TL1A, CD30 or CD30 ligand gene expression in a cell. For example, DR3, TL1A, CD30 or CD30 ligand gene expression can be reduced by inactivating or "knocking out" the DR3, TL1A, CD30 or CD30 ligand gene or its promoter using targeted homologous recombination (see, for example, Kempin et al., Nature 389: 802, 1997; Smithies et al., Nature 317:230-234, 1985; Thomas and Capecchi, Cell 51:503-512, 1987; and Thompson et al., Cell 5:313-321, 1989). For instance, a mutant, non-functional DR3, TL1A, CD30 or CD30 ligand gene variant, or a completely unrelated DNA sequence, flanked by DNA homologous to the endogenous DR3, TL1A, CD30 or CD30 ligand gene, (either the coding regions or regulatory regions of the DR3, TL1A, CD30 or CD30 ligand gene) can be used, with or without a selectable marker and/or a negative selectable marker, to transfect cells that express DR3, TL1A, CD30 or CD30 ligand protein, respectively, *in vivo.*

DR3, TL1A, CD30 or CD30 ligand gene expression can also be reduced by targeting deoxyribonucleotide sequences complementary to the regulatory region of the DR3, TL1A, CD30 or CD30 ligand gene, that is, the DR3, TL1A, CD30 or CD30 ligand promoter and/or enhancers, to form triple helical structures that prevent transcription of the respective in target cells (see generally, Helene, C., Anticancer Drug Des. 6(6):569-84, 1991; Helene, C., et al., Ann. N.Y. Acad. Sci. 660:27-36, 1992; and Maher, L. J., Bioassays 14(12):807-15, 1992). Nucleic acid molecules to be used in this technique are preferably single-stranded and composed of deoxyribonucleotides.

In addition to the foregoing, RNAi can be used to down-regulate DR3, TL1A, CD30 or CD30 ligand expression in a cell. RNAi is a method of interfering with the transcription of specific mRNAs through the production of small RNAs (siRNAs) and short hairpin RNAs (shRNAs) (see Paddison and Hannon, Cancer Cell 2:17-23, 2002; Fire et al., Nature 391:806-811 (1998); Hammond et al. Nature Rev Gen 2: 110-119 (2001); Sharp, Genes Dev 15: 485-490 (2001); Hutvagner and Zamore, Curr Opin Genetics & Development 12:225-232 (2002); Bernstein et al., Nature 409:363-366 (2001); Nykanen et al., Cell 107:309-321 (2001)).

Methods of decreasing an activity of a polypeptide, for example, DR3 or CD30, are well known to those skilled in the art. It is understood that a decrease in activity of a polypeptide includes both decreasing the expression level of the polypeptide as well as decreasing a biological activity exhibited by the polypeptide.

A DR3 or CD30 activity can also be decreased using an inhibitor. An inhibitor can be a compound that decreases expression, activity or intracellular signaling of DR3 or CD30. Such an inhibitor can be, for example, a small molecule, protein, peptide, peptidomimetic, ribozyme, nucleic acid molecule or oligonucleotide, oligosaccharide, or combination thereof, as disclosed herein. Methods for generating such molecules are well known to those skilled in the art (Huse, U.S. Patent No. 5,264,563; Francis et al., Curr. Opin. Chem. Biol. 2:422-428 (1998); Tietze et al., Curr. Biol., 2:363-371 (1998); Sofia, Mol. Divers. 3:75-94 (1998); Eichler et al., Med. Res. Rev. 15:481-496 (1995); Gordon et al., J. Med. Chem. 37: 1233-1251 (1994); Gordon et al., J. Med. Chem. 37: 1385-1401 (1994); Gordon et al., Acc. Chem. Res. 29:144-154 (1996); Wilson and Czarnik, eds., Combinatorial Chemistry: Synthesis and Application, John Wiley & Sons, New York (1997)). Libraries containing large numbers of natural and synthetic compounds also can be obtained from commercial sources. Combinatorial libraries of molecules can be prepared using well known combinatorial chemistry methods, as discussed above. An inhibitor can include, for example, an antagonist; a dominant negative molecule that prevents activation of DR3 or CD30; antibodies, proteins, small molecules and oligonucleotides that inhibit an activity or expression of DR3 or CD30; ribozymes, antisense nucleic acid molecules, and nucleic acid molecules encoding negative regulatory transcription factors that prevent or reduce DR3 or CD30 expression, as well as cells or viruses containing such ribozymes and nucleic acid molecules. One skilled in the art will readily understand that these and other molecules that inhibit DR3 or CD30 expression, activity or signaling can be used as an inhibitor.

One skilled in the art can readily determine a decrease in activity or expression of a DR3 or CD30. For example, nucleic acid probes or primers can be used to examine expression of DR3 or CD30 mRNA, and DR3 or CD30 antibodies can be used to examine expression levels of the respective polypeptides. The effect of an inhibitor can be readily determined by assaying its effect on a biological activity, for example, expression ofIL-13. These and other suitable methods, which can be readily determined by those skilled in the art, can be used to test the effect of a compound as a potential inhibitor of DR3 or CD30.

Other methods of modulating DR3 or CD30 function can also be used, for example, modulating a signaling function of DR3 or CD30. For example, a method for modulating a CD30 function is to interfere with downstream signaling initiated through CD30. For example, CD30 signaling is mediated by TRAF2 and p38. Agents that target these molecules might be used to modulate CD30 function. Pharmacologic inhibitors of p38 are known. Agents capable of modulating TRAF2 and p38 function can be made according to known techniques, for example, anti-sense or RNAi constructs.

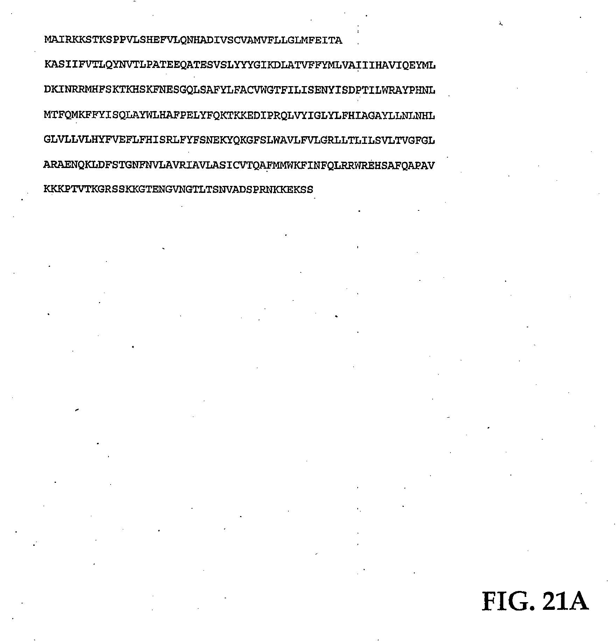

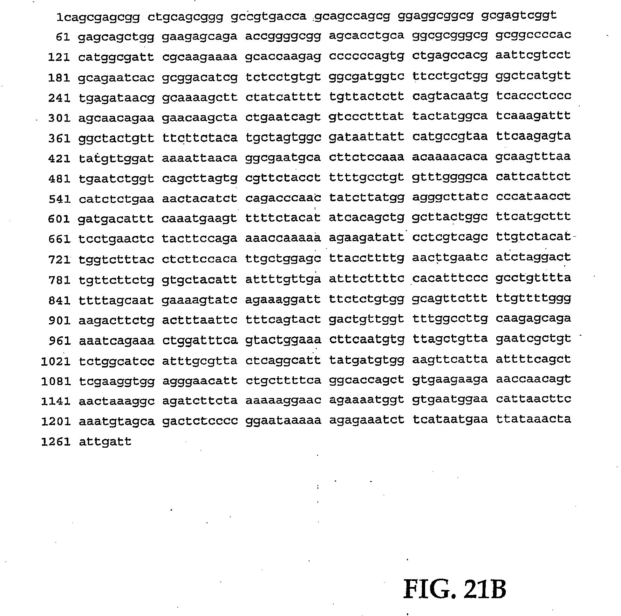

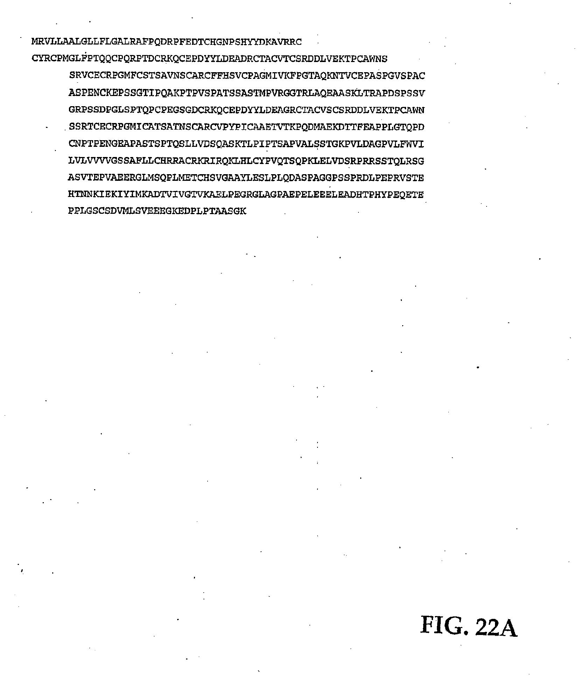

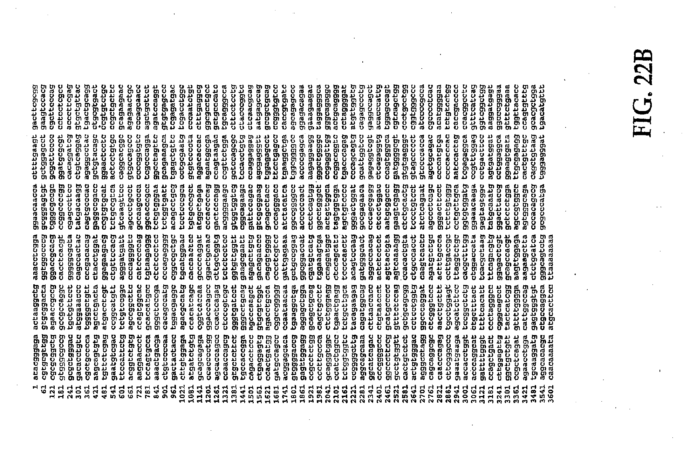

As described in the Examples, a mouse model has been used to identify agents that modulate DR3 and CD30 expression and/or activity and to examine the role of DR3 and CD30 signaling in IL-13 production. However, it is understood by those skilled in the art that such a model is considered representative of other animal models, including human. In such a case, one skilled in the art can readily determine a suitable form of an agent for a particular organism. For example, the form of an agent that functions in a mouse and modulates DR3 or CD30 can be used in a human if that form has substantially the same modulating activity in a human. Alternatively, an analogous human form of the agent can be readily generated by one skilled in the art using, for example, the human sequence of DR3 or CD30 (see Figures 21 and 22). For example, a soluble form of DR3 or CD30 that functions as a dominant negative can be generated from the human sequences of DR3 and CD30 using methods well known to those skilled in the art. The use of the human form can be useful for limiting undesirable immune responses against a foreign antigen. Similarly, a humanized form of an antibody, including a grafted antibody using CDRs from a non-human antibody, for example, mouse, hamster, rabbit, and the like, can be used to treat a human so long as the grafted form has sufficient affinity and specificity for the human form of the antigen. If the DR3 or CD30 target molecule is human, the human sequence can be used to test the effectiveness of an agent in modulating the activity of the human form of DR3 or CD30. For example, the human sequence can be used to screen for antibodies that bind to the respective DR3 or CD30 molecules, or an antibody generated against a DR3 or CD30 molecule of another species can be used if the antibody cross-reacts with the human DR3 or CD30 and binds with sufficient affinity and specificity. One skilled in the art can readily determine a suitable form of an agent of the invention for a particular need.

### Modulating DR3 Function by Overexpression of DR3 Transcripts or by Selectively Expressing Certain Splice Variants of DR3 Transcripts

Because DR3 initiates dominant Th2 polarization, increasing DR3 activity will be beneficial in autoimmune syndromes dominated by Th1 activity. These include multiple sclerosis, rheumatoid arthritis and others. In such cases, DR3 activity can be upregulated by overexpressing a DR3 transcript or by selectively expressing certain splice variants of DR3 transcripts.

### Modulation of PKC activity

As disclosed herein, PKC activation mediates correct splicing of DR3 and can therefore be used to modulate DR3 expression. PKC activity can be increased or decreased using known agents. To reduce PKC activity, agents that might be used include PKC inhibitor peptide (Upstate Biotechnology), H7, Bryostatin, GF109203X (Bisindolymaleimide), RO 318220, myristolated EGF-R fragment, RO 32-0432, and staurosporin. To enhance PKC activity, agents that might be used include phorbol esters such as PMA.

### Methods of Delivering an Agent to a Cell

Agents can be delivered to a cell by any known method. For example, a composition containing the agent can be added to cells suspended in medium. Alternatively, an agent can be administered to an animal, for example by a parenteral route, having a cell expressing DR3, TL1A, CD30 or CD30 ligand so that the agent binds to the cell *in situ.*

### Modulating DR3 or CD30 Function in an Animal Subject

The agents described above may be administered to animals including human beings in any suitable formulation. For example, compositions for targeting a DR3-expressing or CD30-expressing cell may be formulated in pharmaceutically acceptable carriers or diluents such as physiological saline or a buffered salt solution. Suitable carriers and diluents can be selected on the basis of mode and route of administration and standard pharmaceutical practice. A description of exemplary pharmaceutically acceptable carriers and diluents, as well as pharmaceutical formulations, can be found in Remington's Pharmaceutical Sciences, a standard text in this field, and in USP/NF. Other substances may be added to the compositions to stabilize and/or preserve the compositions.

When administered to a subject, a composition can be administered as a pharmaceutical composition containing, for example, a pharmaceutically acceptable carrier. Pharmaceutically acceptable carriers are well known in the art and include, for example, aqueous solutions such as water or physiologically buffered saline or other solvents or vehicles such as glycols, glycerol, oils such as olive oil or injectable organic esters. A pharmaceutically acceptable carrier can contain physiologically acceptable compounds that act, for example, to stabilize or to increase the absorption of the composition. Such physiologically acceptable compounds include, for example, carbohydrates, such as glucose, sucrose or dextrans, antioxidants, such as ascorbic acid or glutathione, chelating agents, low molecular weight proteins or other stabilizers or excipients. One skilled in the art will know that the choice of a pharmaceutically acceptable carrier, including a physiologically acceptable compound, depends, for example, on the route of administration of the composition. One skilled in the art will know that a pharmaceutical composition can be administered to a subject by various routes including, for example, orally or parenterally, such as intravenously, intramuscularly, intraperitoneally, or by inhalation. The composition can be administered by injection or by intubation.

### Polarizing a T Cell Response/Asthma and Other Disorders

Polarizing a T cell response toward a Th1 or Th2 pathway by modulating DR3 activity should be useful for treating a number of diseases. For example, suppressing Th2 responses with DR3 blockers should be helpful for treating asthma and for the immunotherapy of tumors. Enhancing Th2 responses with DR3 agonists, on the other hand, should be beneficial for treating Th1-dominated autoimmunity and for reducing the risk of transplant rejection.

As disclosed herein, inhibiting both DR3 and CD30 activity synergistically inhibits IL-13 signaling (see Example 8). There are additionally described using one or more agents to decrease the activity of both DR3 and CD30. Since inhibiting DR3 and CD30 activity decreases IL-13 expression, it is understood that a method can use a combination of the compositions disclosed herein to decrease both DR3 and CD30 activity. Such a combination can act synergistically to decrease IL-13 expression. Such a combination can therefore be used to treat an inflammatory lung disease such as asthma.

Allergic asthma (airway hyper reactivity) is caused by air way exposure to an antigen of an individual who has been sensitized to the same antigen by previous exposure. The airway associated (mucosal) immune system responds to antigenic challenge with IL-13 production, which sets into motion the sequelae of airway hyper reactivity.

As disclosed herein, DR3 is expressed on NKT cells, and the DR3 ligand TL1A is expressed in bronchial lymph nodes (Example 9). Without being bound by a particular mechanism, the experimental data support the following model for the pathogenic events leading to asthma. Antigen exposure through the airways results in the uptake of the antigen (exemplified with ovalbumin in studies disclosed herein) by dendritic cells (DC) located in the mucosa and submucosa. Antigen loaded dendritic cells become activated and migrate to draining lymph nodes, where they express TL1A on their surface. Lymph nodes contain NKT cells among their residents. NKT cells constitutively express DR3 and are susceptible to TL1A signals by antigen loaded DC arriving in the lymph node. NKT respond to DR3 triggering with IL-13 production. This event recruits local antigen specific memory-CD4 T cells to the dendritic cells and mediates CD4-clonal expansion and increased TH2 cytokine production. Clonal expansion of the memory CD4 T cells is enhanced by CD30 signals emanating on activated CD4 cells through CD30-L binding.

Blockade of TL1A/DR3 inhibits the initiating (triggering event) while CD30/CD30-L blockade inhibits the amplification phase (CD4 clonal expansion). TL1A and DR3 may also be involved in amplification. Both the initiation and amplification contribute to the full blown manifestation of asthma.

There are described methods of screening for an agent that modulates DR3 or CD30 signaling, for example, inhibiting a DR3 or CD30 activity such as IL-13 production. Such an agent can be screened by the methods disclosed herein. Thus, there are described methods for identifying drug candidates for the treatment of inflammatory lung diseases, including asthma. There is described a method of identifying an antibody that specifically binds to DR3 or CD30. The antibody can be generated using routine methods, as disclosed herein (see Example 4). In a particular embodiment, the antibody is generated against the human DR3 or CD30 sequence (see Figures 21 and 22). Other types of agents, as disclosed herein, can also be identified.

### EXAMPLES

### EXAMPLE 1

### Generation and Characterization of DR3 Transgenic Mice

Materials and methods: Mice. All mice were used at 6-12 weeks of age and were maintained in pathogen-free facilities in accordance with the guidelines of University of Miami Animal Care and Use Committee.

Media and Reagents. Cells were cultured in Iscove's Modified Dulbecco's Minimal Essential Medium (Invitrogen, Carlsbad, CA) supplemented with 10% heat-inactivated FBS (Invitrogen), 10µg/ml gentamycin (Invitrogen), and 50µM β-mercaptoethanol (Bio-Rad). Monoclonal anti-mouse CD3 and anti-human CD3 were purified from culture supernatants of the 2C11 and the OKT3 cell lines, respectively (ATCC, Manassas, VA). Monoclonal anti-mouse CD28 and anti-human CD28 were purchased from eBioscience (San Diego, CA). Concanavalin A (ConA), phytohemagglutinin (PHA), and lipopolysaccharide (LPS) were from Sigma (St. Louis, MO). Recombinant murine IL-2 was from BioSource International (Camarillo, CA). Phorbol-12-myristate-13-acetate (PMA) and ionomycin were purchased from Calbiochem (San Diego, CA).

Antibodies. Directly conjugated monoclonal antibodies, including fluorescein isothiocyanate (FITC) and Cychrome-conjugated anti-mouse CD4, phycoerythrin (PE) and Cychrome-conjugated anti-mouse CD8a, FITC-conjugated anti-mouse B220, FITC-conjugated anti-mouse CD25, PE-conjugated Annexin and 7-amino actinomycin (7-AAD) were purchased from BD/PharMingen (San Diego, CA). Hamster IgG control was purchased from eBioscience. Prior to staining, cells were treated with purified anti-mouse CD16/CD32 (Fc-γIII/II receptor, PharMingen) and purified human IgG (Jackson ImmunoResearch, West Grove, PA).

Generation of Armenian hamster anti-mouse DR3 and anti-mouse TL1A monoclonal antibodies. The extracellular portion of mouse DR3 was cloned in frame with the Fc part of mouse IgG1 into the modified expression vector pBMG-Neo, and the construct was transfected into a NIH 3T3 fibroblast cell line using CaPO₄ precipitation. Positive clones were selected with G418, recloned and tested for production of mDR3-Ig by ELISA. MDR3-Ig was purified from the serum-free supernatant of transfected cells on a protein A column, dialyzed into PBS and filter-sterilized. Cloned mTL1A-maltose binding protein (MBP) was expressed in E. *coli* and the fusion protein purified on a maltose-agarose column.

Armenian hamsters were immunized three times biweekly with 50 µg of mDR3-Ig or mTL1A-MBP in Freund's adjuvant intraperitoneally. Three days prior to the fusion, hamsters were boosted with 50 µg of the proteins intravenously. Hamster splenocytes were fused with the murine myeloma SP20 with polyethylene glycol (PEG) and then plated in methylcellulose-based medium for two weeks (ClonaCell-HY kit, StemCell Technologies Inc., Vancouver, Canada). One thousand colonies were picked up and analyzed by ELISA in plates coated with the immunizing fusion protein. Supernatants from positive clones were tested for the ability to detect mDR3 isoforms in transfected cells by flow cytometry and western blotting. Antibodies were purified from a Nutridoma-SP (Roche, Indianapolis, IN) supernatant on a protein G column, dialyzed into PBS and filter sterilized.

Flow cytometry analysis. Single cell suspensions were prepared from thymus, spleen, or inguinal lymph nodes. 10⁵ cells were stained with CD4-FITC, CD8-Cyc, and Armenian hamster anti-mouse DR3 or anti-mouse TL1A for 30 minutes at 4°C. Cells were washed in FACS buffer (PBS containing 0.5% BSA and 2mM EDTA) and then treated with human IgG for 5 min at 4°C, before staining with goat anti-Armenian hamster IgG-Biotin (Jackson ImmunoResearch) for 30 minutes at 4°C. Cells were washed in FACS buffer and then stained with Streptavidin-PE (PharMingen) for 30 minutes at 4°C. Samples were analyzed using a Becton Dickinson fluorescence activated cell sorter (FACS) LSR instrument (Becton Dickinson; San Jose CA) and CELLQuest^{™} software. B220-FITC was also combined with Armenian hamster anti-mouse DR3 or anti-mouse TL1A antibodies to detect their expression level in B cells.

RT-PCR. Messenger RNA was extracted from murine cell lines or tissues with the Micro Fast-Track kit (Invitrogen) and cDNA was reverse transcribed using the Superscript II kit (Invitrogen). RT-PCR products were sub-cloned into the PCR II vector using the TOPO cloning kit (Invitrogen) and were confirmed as splice forms of mDR3 by sequencing.

Activation-induced alternative splicing of DR3 was studied with human cells because splicing products could be separated after PCR by agarose gel electrophoresis. Human PBMCs were isolated from healthy donors by Ficoll Hypaque density gradient centrifugation. 5 million cells per sample were activated with PHA (5µg/ml), or immobilized anti-hCD3 (OKT3, 5µg/ml) and anti-hCD28 (1µg/ml), or PMA (10ng/ml) and ionomycin (400ng/ml). The cells were harvested at the indicated time points and mRNA extracted and converted to cDNA using the Invitrogen kit. Human *β*-actin was used as internal control. Quantitation of PCR products was done with the aid of Molecular Analyst software (BioRad).

Generation of transgenic mice. The full-length molecule of murine DR3 (mDR3-FL) and the DR3 splice variant lacking the 5th and 6th exons (mDR3-Δ5,6) and the dominant negative version of DR3, mDR3-DN, aa 1-234, lacking the intracellular domain were cloned into the EcoR I and BamH I sites of human CD2 promoter and enhancer vector (Love et al., J Exp Med 179:1485, 1994). DNA fragments to be injected into oocytes were separated from the vector sequences by Not I digestion and purified by Gel purification (Qiagen, Valencia, CA), and elution (Schleicher & Schuell, Keene, NH). Microinjections of DNA into the fertilized eggs were done by the transgenic facility at the University of Miami, School of Medicine. Potential founders were screened by PCR from tail DNA. The primer pair was located upstream and downstream of the cloning sites, therefore the same primer pair was used for the three mDR3 transgenes. The upstream primer is 5' CGC TCT TGC TCT CTG TGT ATG 3' (SEQ ID NO:5) and the downstream primer is 5' CTG CCA GCC CTC TTC CAT C 3' (SEQ ID NO:6). Transgenic mice were bred into the C57BL/6J background by serially mating hemizygous transgenic animals with w.t. C57BL/6J (Jackson Laboratories, Bar Harbor, ME).

T cell proliferation assay. Splenocytes were plated in triplicate at 1 x 10⁵ cells/well in 96-well flat-bottomed plates. Cells were activated with immobilized anti-CD3 (2µg/ml) with or without soluble anti-CD28 (1µg/ml), or ConA (5µg/ml) or PMA (10ng/ml) with ionomycin (400ng/ml). For T cell proliferation, purified CD4+ cells at 1 x 10⁵ cells/well or CD8+ cells at 5x 10⁴ cells/well were stimulated with coated anti-CD3 (2µg/ml) with soluble anti-CD28 (1µg/ml). Recombinant mIL-2 was added to the culture at 1000U/ml in indicated experiments. Cells were cultured for 72 hr and pulsed for the last 6 hr incubation with 1µCi/well of [³H] thymidine (Perkin Elmer, Boston, MA), and thymidine incorporation was quantitated using a scintillation counter.

Preparation of purified CD4+ and CD8+ cells. Murine CD4+ or CD8+ or T cells were purified from splenocytes by negative selection (SpinSep kit by StemCell Technology Inc.) according to the manufacturer's protocol. The purity was routinely around 90%-96% examined by staining with CD4-Cyc or CD8-PE.

Immunization and antibody isotype. Adult (6-10 wk old) transgenic and w.t. mice were immunized with 100µg dinitrophenyl (DNP) -conjugated keyhole limpet hemocyanin (DNP-KLH) (CalBiochem). Each mouse was injected at three sites, i.p. and s.c. between the shoulder blades, and at the base of the tail with 100µl/site in sterile PBS. One week and three weeks after immunization, mice were bled and serum was separated for ELISA analysis of anti-DNP specific IgG1 and anti-DNP-specific IgG2a antibodies.

Cytokine and serum ELISA. For cytokine ELISA assays, supernatants were collected during the proliferation assay. Sandwich ELISA was performed per the manufacturer's instructions. Antibody pairs from BD were used for IL-2, IFN-γ, and IL-4 analysis. Reagents for IL-13 ELISA were purchased from R&D Systems (Minneapolis, MN) and reagents for IL-5 ELISA were purchased from eBioscience.

To determine the isotype of anti-DNP-specific IgG1 and IgG2a antibodies, sera from individual animals were analyzed. 96-well plates were coated with 0.8µg/ml DNP-albumin (DNP-BSA) (CalBiochem) overnight at 4°C. The wells were then blocked with PBS containing 10% FBS (blocking buffer) for 1 hr at room temperature. The plates were washed with PBS containing 0.05% Tween-20 (wash buffer). Serum was serially diluted in blocking buffer and incubated at room temperature for 2 hrs. The plates were washed and 100µl of biotin-conjugated anti-mouse IgG1 or biotin-conjugated anti-mouse IgG2a at 2µg/ml (both from BD/PharMingen) was added to each well and incubated for 1 hr at room temperature. The plates were washed and 100µl of 1:1000 dilution of Streptavidin-horseradish peroxidase (HRP) (BD/PharMingen) was added to each well for 30 minutes at room temperature. The plates were washed again and 100µl of 2,2'-azinobis-[3-ethylbenzothizoline-6-sulfonic acid], diammonium salt (ABTS) substrate solution was added into each well. The plates were read on an ELISA reader (Benchmark Plus, Bio-Rad).

Statistical analyses. Statistical analyses using a two-tailed Student's t test were performed with the GraphPad Prism Software, San Diego, CA; p<0.05 is considered significant. Data in the text are presented as the mean ± SEM.

Results: Expression of mDR3 and mTL1A in lymphoid compartments. Monoclonal antibodies to murine DR3 and TL1A were generated by immunizing Armenian hamsters, using a mDR3-Ig fusion protein or a mTL1A-MBP fusion protein as antigen. Splenocyte fusion with the murine myeloma SP20 and HAT selection generated the hybridomas. Antibody specificity of hybridoma supernatants was evaluated by ELISA using the fusion proteins to coat microliter plates and by flow cytometry of hybridoma supernatant binding to cell lines transfected with mDR3 or mTL1A.

Because mDR3 protein was expressed at very low levels in resting lymphocytes, a three-layer sandwich staining assay was developed to amplify the signal. In the thymus, the expression of mDR3 was restricted to single positive CD4+ and CD8+ populations and absent in double positive or double negative thymocytes (Figure 1A). In spleen and lymph nodes, the expression of mDR3 was restricted to CD4+ and CD8+ T cells with higher expression in CD4+ cells, and was not observed in B cells and other non-T cells (Figure 1B). Murine TL1A was not detectable in resting spleen cells (Figure 1B), thymocytes or lymph node cells. However, both mDR3 and mTL1A were induced after 24 hrs CD3 activation of CD4+ and CD8+ cells, but not in LPS-activated B cells (Figure 1C).

Murine DR3 has ten forms of alternatively spliced mRNA. Human DR3 has been reported to exist in 12 differentially spliced mRNA forms, raising the possibility that similar molecular pathways have been conserved evolutionarily. By performing RT-PCR on mouse cell lines and mouse tissues, 10 splice forms for mDR3 were identified (Figure 2A). Four splice forms retain the second intron, thereby creating an in frame stop codon that would cause early translation termination, most likely resulting in a non-functional protein. Two splice forms lacking exon 5 or exon 6 encode two potentially soluble proteins with only three complete cysteine rich domains (CRDs) (Wang et al., Immunogenetics 53:59, 2001). Three forms missing both exon 5 and 6 encode transmembrane receptors lacking the fourth CRD.

To address the effect of T cell activation on human DR3 splicing, a RT-PCR assay was developed. Because seven out of the twelve splice forms of hDR3 skip exon 6, the exon right before the transmembrane domain, primer pairs that located in exons 4 and 7 were designed to focus the study around exon 6. In resting human T cells, three major splice forms were readily resolved and were expressed at nearly equivalent levels. After activation by PHA, or anti-hCD3 and anti-hCD28, or PMA and ionomycin, the full-length form of DR3 was induced to twice the level of the other two forms. Upregulation of the full-length mRNA of DR3 is an early event (Figures 2B, 2C), being detectable already after three hrs. Splicing of DR3 is independent of new protein synthesis but requires PKC signals as indicated by pharmacological blockers.

Expression of transgenic DR3 in full-length form, as transmembrane splice variant and as dominant negative form. One splice form of DR3, designated as mDR3-Δ5, 6 lacks exons 5 and 6 but retains the reading frame; it encodes a transmembrane protein with a typical death domain, but lacking the fourth CRD. Whether this form might differ from full-length DR3 by ligand binding specificity or affinity was investigated using transgenic lines for both mDR3-FL (full-length) and mDR3-Δ5, 6 in addition to a mDR3-DN (dominant negative) transgene to mimic the phenotype of knockout mice. Expression of these three forms was directed by the human CD2 promoter and enhancer (Figure 3A). DR3 was overexpressed in all transgenic founders and the expression was T cell-specific (Figure 3B).

Reduction of CD8 T cells and CD4 T cells in DR3 transgenic mice. Five mice derived from each of two DR3-FL transgenic founder mice, five mice from each of two mDR3-Δ5,6 transgenic founder mice and five mice from a non-transgenic littermate were analyzed to determine the frequency of lymphocyte subpopulations in lymphoid organs (Figure 4). The total number of thymocytes and splenocytes in DR3 transgenic mice tended to be lower, although the difference was not significant in most cases. In lymph nodes, however, the total cell number was significantly diminished in DR3-FL transgenic mice and in the offspring of one of the founders of the DR3-Δ5,6 tg mice. Analyzing the number of CD4 and CD8 T cells, a strong reduction of CD8 T cells by 50% or more was found in lymph nodes, spleen and thymus. The number of CD4 T cells was less affected. DR3-Δ5,6-tg CD4 cells were normal in lymph nodes and spleen but reduced in the thymus. DR3-FL-tg CD4 cells on the other hand were affected and reduced in number in all three organs. The data indicate that transgenic overexpression of DR3 is more detrimental to CD8 cells than CD4 cells, and the DR3-FL transgene is more effective in this sense than the Δ-5,6 transgene. The DR3-DN transgene had no significant effect on the number of either CD4 or CD8 cells or the cellularity of any of the lymphoid organs.

Impaired activation-induced proliferation in DR3-transgenic mice. Splenocytes from mDR3-Δ5,6-tg and mDR3-FL-tg showed a dramatic reduction of proliferation in response to anti-CD3 with or without anti-CD28 or to Con A alone when compared to littermate controls (Figure 5A). In contrast, splenocytes from mDR3-DN-tg proliferated at a comparable level as the littermate control cells, suggesting that DR3 signals do not contribute to proliferation on w.t. cells. To exclude the possibility that diminished proliferation was due to lower T cell numbers in splenocytes, CD4+ and CD8+ cells were purified by negative selection and analyzed. Both, transgenic CD4+ and CD8+ cells proliferated poorly in response to anti-CD3 and anti-CD28. The effect of the FL and Δ5,6 DR3 transgenes was similar (Figure 5B,C). However, transgenic T cells were able to proliferate normally in response to PMA and ionomycin, indicating that the DR3 transgenes interfered with signaling rather than with the cell cycle. Diminished thymidine uptake by transgenic T cells was not due to increased apoptosis; transgenic CD4+ cells underwent apoptosis at a comparable level as littermate control cells as determined by Annexin and 7-AAD staining (Figure 5D). Transgenic CD4+ and CD8+ T cells upregulated IL-2Rα (CD25) as well as the littermate control cells, implying that the proliferation defect was not due to unresponsiveness to IL-2 (Figure 5E). It was not observed, however, that transgenic T cells produced less IL-2 compared to control T cells (Figure 5F). Nonetheless, added exogenous IL-2 did not rescue the proliferation defect of DR3 transgenic cells (Figure 5B).

DR3-transgenic CD4+ cells spontaneously polarize towards Th2 lineage commitment *in vitro* and *in vivo.* DR3 transgenic CD4+ cells upon activation spontaneously differentiated into Th2 cells without being subjected to Th2 polarizing conditions. After a three-day activation period with immobilized anti-CD3 and soluble anti-CD28, DR3 transgenic CD4+ cells produced significantly higher amounts of IL-4, IL-5, and IL-13 than control CD4+ cells. Under the same conditions, IFN-γ, the signature cytokine for Th1 cells, was reduced in mDR3-FL-tg cells but not diminished in DR3-Δ5,6-transgenic cells when compared to control non-transgenic CD4+ cells. The DR3-DN transgene had no effect on IFN-γ or IL-4 production (Figure 6A).

DR3 transgenic CD4+ T cells, while exhibiting diminished proliferation, produced significantly higher amounts of IL-4 after 24-hour and 48-hour activation; at the same time points, control CD4+ T cells produced no detectable or minute amounts of IL-4 (Figure 6B). Compared to control CD4+ cells, DR3 transgenic CD4+ cells consistently produced lower amounts of IL-2. Production of IFN-γ was normal in DR3-Δ5,6-tg and diminished in DR3-FL-tg CD4 cells.

Th2 type cytokines, especially IL-4, promote IgG1 antibody production by B cells; on the other hand, Th1 type cytokines such as IFN-*γ* promote IgG2a antibody production by B cells. The impact of overexpression of mDR3 in transgenic mice was measured *in vivo* by immunizing mice with DNP-KLH and analyzing levels of anti-DNP-specific IgG1 and IgG2a antibodies. Before immunization, DR3-Δ5,6 transgenic mice contained comparable levels of serum IgG1, IgG2a, IgG2b, and IgE as control mice (Figure 7A). One and 3 weeks after immunization, DR3 transgenic mice generated two-fold higher titers of antigen-specific IgG1 than littermate controls, while they generated comparable levels of antigen-specific IgG2a (Figure 7B). The levels of anti-DNP-specific IgE were not detectable. In agreement with *in vitro* cytokine production, mDR3-DN-tg maintained comparable levels of antigen specific responses as the littermate mice.

### EXAMPLE 2

### DR3 Transgenic Mouse Model of Lung Inflammation

DR3 and TL1A expression in activated lymphocytes. DR3 mRNA exists in randomly spliced forms in resting lymphocytes. Small amounts of DR3 protein are found on resting CD4 and CD8 cells. No TL1A mRNA is detected in resting cells from adult mice or human beings. When activated with anti-CD3 and anti-CD28, full-length DR3 mRNA and protein is upregulated rapidly in both CD4+ and CD8+ T cells by correct mRNA splicing. T cell activation also results in TL1A protein expression; activated B cells do not express DR3 or TL1A protein. To study mouse DR3 and TL1A expression and signaling on a protein level, monoclonal antibodies were developed using recombinant mDR3-Ig and MBP (maltose binding protein)-TL1A fusion proteins as antigens for immunization and hybridoma screening. Both anti-DR3 and anti-TL1A hybridoma supernatants or purified antibodies can be used for flow cytometry and immunohistochemistry on frozen sections. In addition, the anti-DR3 clone 4C12 displayed agonistic activity, imitating TL1A binding and triggering as demonstrated by killing of DR3 transfected cells and stimulating proliferation of activated T cells *in vitro.* The anti-TL1A clone L4G6 exhibited TL1A blocking activity because it inhibited TL1A-mediated killing of DR3-transfected cells *in vitro* and blocked lung inflammation *in vivo.*

Lymphocytes from DR3 transgenic mice produce large amounts of Th2 cytokines, including IL-13. In order to determine the function of DR3 on peripheral T cells, three different transgenic mouse strains were created: one expressing full length DR3; one expressing a dominant negative form of DR3 (DR3-DN); and one expressing TL1A. All were expressed under the control of T cell-specific CD2 promoter. Two functional isoforms of DR3 receptor differing in the number of cysteine-rich domains in the extracellular region (DR3 fl and DR3 Δ5,6) were tried for transgenic expression. Both displayed almost the identical phenotype.

In attempts to block DR3 signaling *in vivo* and *in vitro,* the dominant negative DR3-DN transgene was created by removal of the cytoplasmic signaling region of the receptor. When overexpressed in T cells, DR3-DN inhibits DR3 signaling, acting as a decoy receptor and by making non-signaling trimers with w.t. DR3 chains. Founders for DR3, DR3-DN and TL1A transgenic mice were screened by tail biopsies, and transgene expression was verified by FACS. DR3-transgene expression was higher in resting transgenic cells than in activated w.t. cells. The expression levels of DR3-DN and TL1A transgenes were similar to that of DR3 transgenes. CD4+ cells from DR3 transgenic mice produced higher amounts of the Th2 cytokines (1L-4, IL-5, IL-13) when activated *in vitro* with plate bound anti-CD3. At the same time, DR3 transgenic CD4+ cells produced significantly decreased amounts of IFN-γ and IL-2, suggesting that transgenic DR3 causes Th2 skewing in mice. The dominant negative DR3 transgene had no effect on cytokine production in primary activation, suggesting that DR3 does not contribute to priming in w.t. cells.

The antibody response to DNP in DR3 transgenic mice was shifted to Th2 type antibodies. Higher levels of DNP-specific IgG1 (Th2) were detected in the serum of DR3 transgenic mice immunized with DNP-KLH, while IgG2a levels (Th1) were similar to w.t. The DR-DN tg did not affect antibody isotype after primary immunization. The finding that DR3-DN and TL1A transgenic cells produced Th1 and Th2 cytokines similar to w.t. cells indicated that the observed effects were not caused by the transgenic construct. To ensure that the phenotype of DR3 transgenic mice was not caused by gene disruption by integrated transgenic construct, litters from different founders were used in experiments.

Because DR3 is a death receptor, potentially capable of inducing cell death via activation of caspases as well as of protecting cells from apoptosis through activation of the NF-κB pathway, the viability of the activated cells was tested with 7-AAD staining. Freshly isolated or activated DR3 transgenic lymphocytes had the same percentage of dead cells when compared to w.t. lymphocytes. DR3 signaling is required for inflammatory lung disease upon airway exposure to antigen. The increased production of the Th2 cytokines IL-4, IL-5 and IL-13 and the Th2 polarization of DR3 transgenic T cells suggested an increased susceptibility to asthma in DR3 transgenic mice.

To test this hypothesis, the mouse model of ovalbumin-induced acute lung inflammation was utilized. Wild type (w.t.), DR3 transgenic and DR3-DN transgenic mice were sensitized with intraperitoneal injections of ovalbumin with alum on days 0 and 5, and challenged with aerosolized ovalbumin on day 12. Three days later, a moderate pulmonary inflammation was observed in w.t. mice. Infiltrating cells representing mostly eosinophils were found in the bronchoalveolar lavage fluid (BALF) (Figure 8A) and in haematoxylin-eosin (H&E) stained sections; mucus hypersecretion was detected with periodic acid-Schiff (PAS) staining (Figure 9, lower row). DR3 transgenic mice had a strongly increased asthmatic phenotype with large numbers of infiltrating cells, more than 90% of which were eosinophils. Mucus secretion was also enhanced (Figure 9), and higher levels of ovalbumin-specific IgE were detected in the serum of DR3 transgenic mice sensitized and challenged with ovalbumin (Figure 8B). IL-4, IL-5 and IL-13 were readily detectable in the BALF of ovalbumin sensitized and challenged DR3 transgenic mice, but barely detectable in BALF from w.t. and DR3-DN transgenic mice. Blockade of DR3 blocks pulmonary inflammation in w.t. mice. In primary activation, DR3-DN transgenic lymphocytes produced w.t. amounts of Th1 and Th2 cytokines when activated by TCR cross-linking *in vitro.* However, DR3-DN transgenic mice sensitized and challenged with ovalbumin showed markedly diminished signs of pulmonary inflammation when compared to w.t. mice (Figures 8, 9). Total cell numbers and eosinophil numbers in BALF were decreased compared to w.t. mice, while the numbers of lymphocytes and macrophages were comparable (Figure 8A). Lung sections from DR3-DN mice also showed significant reduction in eosinophilic infiltration and mucus secretion compared to w.t. mice (Figure 9), and the level of ovalbumin-specific IgE in the serum was significantly decreased (Figure 8B).

Whether blockade of TL1A binding to DR3 *in vivo* blocked lung inflammation was investigated. TL1A blocking antibody L4G6 was administered *in vivo* to ovalbumin-sensitized mice on days -1, 0, +1 and +2 of the airway challenge with aerosol. Blocking of TL1A-DR3 interactions by the antibody resulted in more than 80% reduction of eosinophil numbers in the BALF (Figure 10).

Developmental control of DR3 expression and correlation with neonatal Th2 bias. CD4+ responses to standard, non-polarizing immunization is Th2-skewed in neonates (Figure 11). The level of DR3 expression in resting and activated lymphocytes from adult and newborn mice was compared. Elevated DR3 expression was observed in freshly isolated neonatal CD4+ cells (Figure 12). Neonatal CD4+ cells in the resting state expressed two-fold more DR3 than adult cells based on mean fluorescence intensity (MFI). Activated cells from 7 day old mice expressed maximal DR3 at about 3-4 times the level of activated adult cells. In addition, the kinetics of DR3 expression in 7 day old mice were accelerated compared to adult cells.

DR3 splicing is controlled by PKC activation. Correct splicing of DR3-mRNA is driven by lymphocyte activation. The signals required for DR3 splicing were investigated. Treatment with PMA and Ionomycin (Figure 13) and PMA alone induced correct splicing of DR3, indicating that PKC may be responsible for activation-mediated splicing of DR3. Splicing of DR3 was not blocked by protein synthesis inhibitors. DR3 splicing by PKC was confirmed with pharmacological inhibitors. H7 completely blocked activation-induced splicing of DR3. In contrast the inhibitors of ERK1/2 (UO126, Calbiochem); p38 (SB203580); or Ca-calmodulin dependent CAM-kinase (KN93) had no effect on splicing.

### EXAMPLE 3

### Dominant Negative DR3 Transgene

Blockade of DR3 signals by dominant negative DN-DR3 transgenes on T cells blocks Th2 polarization. CD4 cells were purified by negative selection and were activated with immobilized anti-mouse CD3 (2µg/ml) and soluble anti-mouse CD28 (1µg/ml). Supernatants were collected after a 3-day culture for the primary response. The cells were washed, replated and reactivated with immobilized anti-mouse CD3 (1µg/ml) for two days.

Referring to Figure 14, transgenic full-length FL-DR3 overexpression on T cells caused increased Th2 cytokine production during primary activation. Purified CD4 cells from w.t., (open bar) FL-DR3 transgenic mice (black) and dominant negative DR3 transgenic (gray) mice were activated for three days with anti-CD3 and anti-CD28. After 72h, supernatants were harvested and analyzed (A). The cells were washed and replated on anti CD3 for an additional 48h before analysis of the supernatants (B). Note the different y-axes in secondary activation and increased production in w.t. CD4 but not DN-DR3 tg CD4.

### EXAMPLE 4

### Generation of DR3 and TL1A Antibodies

A DR3-Ig fusion protein was generated, purified and used to immunize hamsters. Hybridoma supernatants were obtained and screened by ELISA using the DR3-Ig fusion protein as a screening agent. The nature of the hybridomas was verified by flow cytometry of DR3 transfected tumor cells, by Western blots, and by functional studies. All of the antibodies detected full-length and alternatively spliced DR3 on transfected cells by FACS, one of the antibodies detected DR3 in Western blots, and one of the antibodies (4C12) displayed agonistic activity, mediating DR3 signaling in the absence of TL1A.

TL1A monoclonal antibodies were obtained by immunizing hamsters with a TL1A-maltose-binding-protein fusion. The TL1A antibodies detected transfected TL1A by flow cytometry. One of the antibodies (L4G6) displayed antagonistic activity, blocking TL1A binding to DR3.

Referring to Figure 15, P815 target cells were transfected with FL-mDR3 or with alternatively spliced mΔ5,6-DR3, a form of DR3 lacking exon 5 and 6 encoding part of the extracellular domain. A. EL4 were transfected with mTL1A and used as effector cells at the indicated effector:target ratio with Cr labeled P815-DR3 or P815-Δ5,6-DR3 in 5 hour assays. B. Supernatants harvested from EL4-TL1A cultures (10⁶/ml, 24h) were used at the indicated concentration with the same P815 targets for 5h and Cr release determined. C. Inhibition of TL1A mediated Cr release by monoclonal antibody L4G6, but not by other antibodies. Clone L2G8 shows partial inhibition. Purified L4G6 antibody causes 50% inhibition at 20ng/ml.

### EXAMPLE 5

### IL-13 Production and Eosinophilia in the Lung

The role of CD30 in lung inflammation was examined using a murine model of AHR induced by immunizing mice with ovalbumin in the presence of alum as adjuvant and two weeks later challenging the mice with ovalbumin through the nasal route or by inhalation of aerosolized ovalbumin (Mattes et al., J. Immunol.167:1683, 2001). Wild-type (w.t.) and CD30 knock out mice were immunized by intraperitoneal (i.p.) injection with ovalbumin (10 µg) and alum (2 mg) on day 0 and day 5. On day 12, the mice were challenged with aerosolized ovalbumin. Control mice (both w.t. and CD30 knockout) were injected with phosphate-buffered saline (PBS) rather than ovalbumin. Three days later, (a) bronchoalveolar fluid (BALF) was collected by lavage (3 x 0.5 ml PBS) (b) the supernatant resulting from homogenized and centrifuging the lungs was collected ("lung fluid"), (c) the thoracic lymph nodes were isolated, and (d) serum was collected. Referring to Figure 16, cellular exudates in the BALF were counted and characterized by Wright Giemsa staining; and IL-13, IL-4, 1L-5, IFN-γ, and GM-CSF levels in the samples were determined by ELISA. The results showed that IL-13 levels in the BALF and lung fluid were lower in the CD30 knockout mice than the w.t. mice. In comparison, however, the levels of IL-4, IL-5, GM-CSF and IFN-γ were about the same in both the CD30 knockout and w.t. mice. Among the ovalbumin-immunized and challenged animals, the number of cells in the BALF was significantly greater for the w.t. animals compared to the CD30 knockout animals. The number of macrophages, lymphocytes, neutrophils, and eosinophils in the BALF was quantified. Although the number of macrophages was about the same in both the w.t. and CD30 knockout mice, the number of the other cells (most notably eosinophils) was markedly decreased in the knockout mice compared to the w.t. mice.

Referring to Figure 17 (left graph), lymphocytes obtained from thoracic lymph nodes of the mice described immediately above were restimulated *in vitro* with ovalbumin and then analyzed for production ofIL-13, IL-4, IL-5, IFN-γ, and GM-CSF. The results showed that IL-13 production was markedly reduced and GM-CSF production was lower in cultures of cells obtained from the CD30 knockout mice compared to those obtained from the w.t. mice. The levels of IL-4, IL-5, and IFN-*γ* were about the same in cultures of cells obtained from both the CD30 knockout and w.t. mice.

Referring to Figure 17 (right), the levels of IgE in the BALF, lung fluid, and serum were determined. The results showed that IgE levels were roughly the same in both the CD30 knockout and w.t. mice.

### EXAMPLE 6

### IL-13 Production by CD30 Signals is Mediated by TRAF2 and p38

CD30 signals are transmitted via TRAF2 and NF-κB. DO11 TCR transgenic mice specific for ovalbumin express high levels of CD30 upon activation. In order to investigate signaling requirements for IL-13 production by CD30, signaling inhibitors and genetically modified transgenic mice were analyzed. TRAF-dominant negative (DN) transgenic-DO 11 TCR transgenic T cells were unable to produce IL-13 upon CD30 signaling; in contrast Iκ-Bα-DN transgenic T cells produced normal levels of IL-13 upon stimulation of CD3 0 with CD30-Ligand (CD153). Similarly pharmacologic p38 inhibitors, but not MEK inhibitors, blocked CD30-mediated IL-13 production. Importantly, referring to Figure 18, CD30 signals are transmitted without concurrent TCR stimulation, unlike CD28 signals that require TCR engagement. Anti-CD30 antibody (Figures 18A, B) or CD30-L alone (C) selectively upregulated IL-13 message and protein, while upregulation of IL-4, IL-5, IL-10 and IFNγ required TCR costimulation.

The role of CD30 in IL-13 production was also investigated using YT cells, a human lymphoma cell line that constitutively overexpresses CD30, Engaging CD30 with the agonistic anti-CD30 antibody C10 caused up-regulation of IL-13 mRNA levels in YT cells. In other experiments, YT cells transfected with TRAF2DN showed down regulation of 95 genes by 1.7 fold or more compared to mock-transfected YT cells. As shown in Figure 19, IL-13 was among the most strongly down-regulated genes. Figure 19 shows gene products grouped by the Gene Spring program in the group of signal transducing molecules, including IL-13.

### EXAMPLE 7

### CD30 Signals Increase MMP9 Production by Lymphocytes

Matrix metalloproteinase 9 (MMP9), a gelatinase, is strongly upregulated by CD30 signals induced with an anti-CD30 agonistic antibody. As shown in Figure 20, this activity was detectable in the supernatant of CD30-activated cells in zymograms. The secretion of MMP9 may be a significant contributor to subepithelial fibrosis via the proteolytic activation of pro-TGF-*β*1 secreted from epithelial cells upon IL-13 stimulation.

### EXAMPLE 8

### EAE Does Not Resolve in CD30-Ligand Knock Out Mice

EAE is known to show spontaneous remission in wild type mice, with a second and third wave of milder disease recurring in a fraction of the affected mice. This undulating form of disease is similar to multiple sclerosis in man. To induce EAE, wild type and CD30-Ligand knock out mice (CD30-LKO) were injected on day 0 with MOG, a major oligodendrocyte glycoprotein-derived peptide under conditions known to induce EAE. The results are shown in Figure 23. Spontaneous resolution of disease did not occur in CD30-L k.o. mice, suggesting that CDD30-L is required for disease resolution.

### EXAMPLE 9

### Anti-CD30 Antibody Interferes with Resolution of EAE in Wild Type mice and Aggravates EAE in CD30-L Knock Out Mice

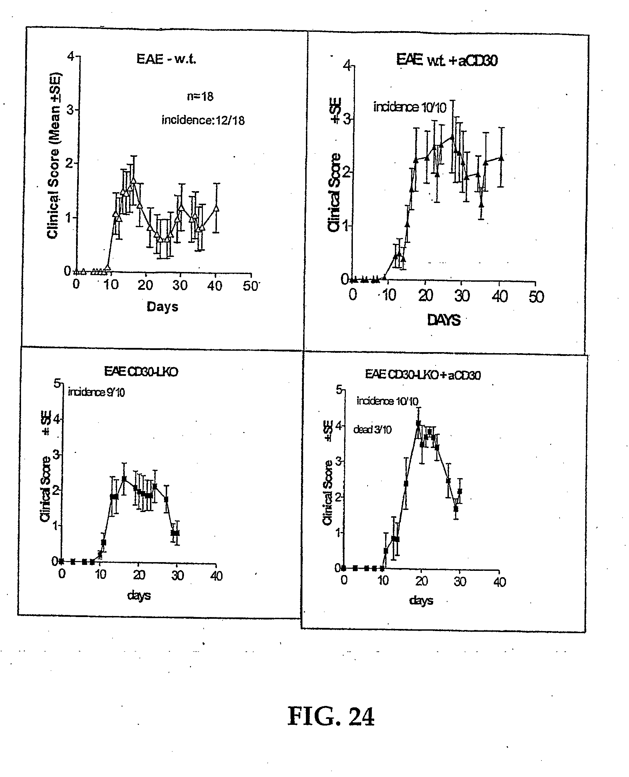

Referring to Figure 24, the effect of anti-CD30 antibody on the resolution of EAE was examined in w.t. mice and CD30-Ligand k.o. mice. Mice were injected with MOG as in Example 8. On days 0, 4, 7 and 12, the mice also received 100 µg anti CD30 antibody (catalog number 558769, BD Biosciences Pharingen, San Diego, CA) intraperitoneally. In the w.t. mice, anti-CD30 antibody administration increased the incidence of disease to 10/10 (100%); caused a more severe form of disease; and prevented resolution of disease (which normally occurs in w.t. mice around day 15). Anti-CD30 antibody treatment therefore imitated the effects seen in CD30-L k.o. mice. Administration of anti-CD30 antibody to CD30-L k.o. mice increased the incidence and severity of disease, and caused lethality in 3 of 10 mice. These data indicate that CD30 is an important negative regulator of immune responses. In the absence of CD30-Ligand or in the presence of anti-CD30 antibody, immune responses are much stronger, indicating that stimulation of CD30 results in down-regulation of the immune response.

### EXAMPLE 10

### Synergy of CD30 and DR3 Blockade in Preventing TH2 Polarization and Asthma

Dominant negative (DN) DR3 transgenic mice are unable to signal via DR3 (see Examples 1-3). These dominant negative DR3 transgenic mice produce diminished TH2 cytokines, including IL-13, and have decreased susceptibility to asthma (Example 2). CD30 deficient mice also show diminished IL-13 production and reduced susceptibility to asthma (see Example 5).

CD30-L deficient mice have been generated that are unable to trigger CD30 signals by the cognate ligand (see Examples 8 and 9). These mice were generated using well known methods and essentially as used to create CD30 deficient mice. CD30-L deficient CD4 T cells have been shown to have diminished Graft versus host Disease Activity following allogeneic bone marrow transplantation.

DN-DR-tg mice are cross bred with CD30-L deficient mice to generate DN-DR3 transgenic, CD30-L deficient mice. Splenocytes from these mice are assayed for T cell proliferation essentially as described in Example 1. The levels of various cytokines, including IL-13, are assayed by ELISA essentially as described in Example 1. The mice are tested for the effect on inflammatory lung disease using the ovalbumin-induced acute lung inflammation model essentially as described in Example 2. These and other types of well known assays can be used to characterize these mice. These mice are expected to have synergistic suppression of IL-13 production and should be even more resistant to asthma than the parental strains.

In another approach, CD30-L mice are treated with a blocking anti TL1A antibody, such as the L4G6 antibody described in Example 4. Cytokines are measured as described above. The effect on signaling is also tested essentially as described in Example 4. This procedure should eliminate TL1A binding to DR3 and synergize with the absence of CD30 signals.

The synergistic effect of blocking CD30 and DR3 signals simultaneously is expected to potentiate the activity of each single agent. Therefore, the use of one or more agents that block both CD30 and DR3 signaling is expected to allow synergistic inhibition of IL-13 signaling, and such a combination can be used to treat inflammatory lung disease, including asthma. The use of agents that block both CD30 and DR3 signaling allows the reduction of the dose of each single agent and diminishes possible side effects while maintaining or increasing therapeutic activity.

### EXAMPLE 11

### Expression of DR3 and TL1A in Cells of the Immune System

The expression of DR3 in specific T cells was examined. DR3 is expressed on natural killer T (NKT) cells. It has been shown by others that NKT cells are required for the induction of asthma.

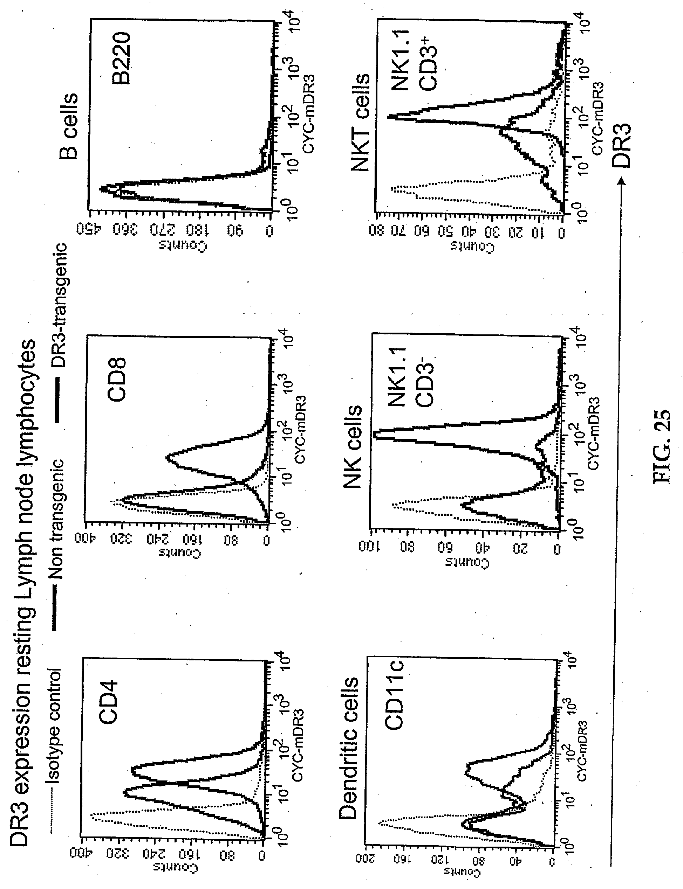

Figure 25 shows expression of mDR3 in lymph nodes of B6 wt mice and DR3 transgenic mice measured by flow cytometry. Resting inguinal lymph node cells were stained with anti DR3 and the respective second antibody. DR3 expression is shown after gating on CD4, CD8, B220 or CD11ccells. NK cells are gated NK1.1 positive and CD3 negative; NKT cells are gated NK1.1 and CD3 double positive cells.

The expression of the DR3 ligand TL1A was also examined. Bronchial lymph nodes, but not other lymph nodes, express TL1A after immunization. Given the expression of TL1A in bronchial lymph nodes and the expression of DR3 on NKT cells, the earliest event in asthma induction can be through bronchial lymph node TL1A binding to DR3 on NKT cells, which get activated to produce IL-13. This is the key event in the initiation of AHR.

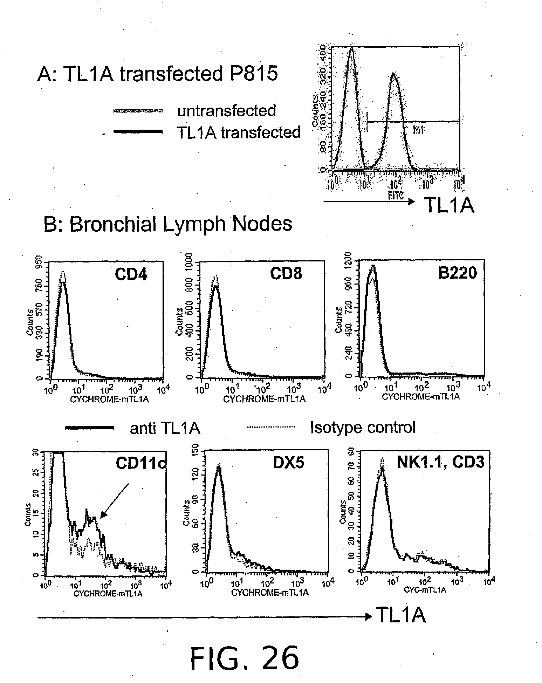

Figure 26 shows expression of mTL1A in bronchial lymph nodes (LNs) of ovalbumin sensitized and aerosol challenged B6 wt mice. Figure 26A shows that anti-mTL1A monoclonal antibody stained mTL1A on TL1A-transfected P815 cells, but not untransfected cells. Figure 26B shows that expression of mTL1A was only detected on a portion of CD11c expressing DCs (arrow) in bronchial lymph node cells from OVA sensitized and aerosol challenged B6 wt mice. Cells were gated on CD4, CD8, B220, CD11c or DX5 positive cells, or NK1.1 and CD3 double positive cells.

CD11c positive DC in other lymph nodes are negative for TL1A. Bronchial lymph nodes are TL1A positive only after aerosol challenege.

## Claims

1. An antibody that specifically binds TL1A for use in a method of treating asthma, wherein the antibody blocks TL1A binding to DR3 and decreases the activity of DR3, whereby IL- 13 expression is decreased.

2. The antibody according to claim 1 for use in a method of treating asthma according to claim 1, wherein the antibody is a monoclonal antibody.

3. The antibody according to claim 1 for use in a method of treating asthma according to claim 1, wherein the asthma is **characterized by** eosinophil infiltration in the lung, and the antibody reduces the number of eosinophils in the lung.

## Patentansprüche

1. Ein Antikörper, der spezifisch an TL1A bindet, zur Verwendung bei einem Verfahren zur Behandlung von Asthma, wobei der Antikörper die Bindung von TL1A an DR3 blockiert und die Aktivität von DR3 mindert und dadurch die Expression von IL-13 verringert.

2. Der Antikörper nach Anspruch 1 zur Verwendung bei einem Verfahren zur Behandlung von Asthma nach Anspruch 1, wobei der Antikörper ein monoklonaler Antikörper ist.

3. Der Antikörper nach Anspruch 1 zur Verwendung bei einem Verfahren zur Behandlung von Asthma nach Anspruch 1, wobei das Asthma durch eosinophile Infiltration der Lunge gekennzeichnet ist und der Antikörper die Zahl der Eosinophilen in der Lunge reduziert.

## Revendications

1. Anticorps se liant spécifiquement à TL1A, à utiliser dans un procédé de traitement de l'asthme, dans lequel l'anticorps bloque la liaison de TL1A à DR3, et réduit l'activité de DR3, ce qui réduit l'expression d'IL-13.

2. Anticorps selon la revendication 1, à utiliser dans un procédé de traitement de l'asthme selon la revendication 1, dans lequel l'anticorps est un anticorps monoclonal.

3. Anticorps selon la revendication 1, à utiliser dans un procédé de traitement de l'asthme selon la revendication 1, dans lequel l'asthme se **caractérise par** une infiltration d'éosinophiles dans le poumon, et l'anticorps réduit le nombre d'éosinophiles dans le poumon.
